# EUROPÄISCHE PATENTSCHRIFT

(11) **EP 0 704 497 B1**
(45) Veröffentlichungstag und Bekanntmachung des Hinweises auf die Patenterteilung: **15.12.1999**
(21) Anmeldenummer: 95810590.0
(22) Anmeldetag: 20.09.1995
(51) Int. Cl.: C09B 67/22, C09B 67/48, C08K 5/3415, C09B 57/00

(54) **Mischkristalle und feste Lösungen von 1,4-Diketopyrrolopyrrolen**
Mixed crystals and solid solutions of 1,4 diketopyrrolopyrroles
Cristaux mixtes et solutions solides de 1,4 dicétopyrrolopyrroles

(30) Priorität: 28.09.1994 CH 293694
(43) Veröffentlichungstag der Anmeldung: 03.04.1996
(73) Patentinhaber: Ciba Specialty Chemicals Holding Inc., 4057 Basel (CH)
(72) Erfinder: Hao, Zhimin, Dr., CH-1723 Marly (CH); Iqbal, Abul, Dr., CH-1732 Arconciel (CH); Medinger, Bernhard, Dr., CH-1735 Giffers (CH); Wallquist, Olof, Dr., CH-1723 Marly (CH)

(56) Entgegenhaltungen:
- EP-A- 0 181 290
- EP-A- 0 184 982
- EP-A- 0 190 999
- EP-A- 0 256 983
- EP-A- 0 492 893
- EP-A- 0 604 370
- EP-A- 0 654 506

## Beschreibung

Die vorliegende Erfindung betrifft neue, einphasige Mischkristalle und feste Lösungen aus zwei unterschiedlichen symmetrischen 1,4-Diketopyrrolopyrrolen und ihre Verwendung als Pigmente.

1,4-Diketopyrrolopyrrole, darunter auch asymmetrische, d.h. des Typs 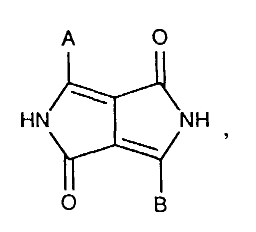 ihre Herstellung und ihre Verwendung als Pigmente, sind z.B. in US-Patent 4 579 949 beschrieben. Aus US-Patent 4 778 899 ist ein Verfahren zur Herstellung reiner asymmetrischer 1,4-Diketopyrrolopyrrole bekannt. Es handelt sich dabei um eine aufwendige Synthese, die über die Stufe spezieller Aminoester oder Pyrrolinone erfolgt.

Aus US-Patent 4 783 540 ist weiterhin bekannt, dass beim Mischen zweier verschiedener 1,4-Diketopyrrolopyrrole, vorzugsweise in einem Verhältnis von 65-90:10-35 Gew.%, und nachfolgende Behandlung, z.B. durch Kneten, Mahlen oder Umfällen, feste Lösungen erhalten werden können. Die festen Lösungen sind durch ihre Röntgenbeugungsdiagramme charakterisiert, wobei sich die Röntgenbeugungsdiagramme der festen Lösungen von der Summe der Röntgenbeugungsdiagramme der Einzelkomponenten unterscheiden. Es ist allerdings festgestellt worden, dass es sich bei den Produkten aller Beispiele ausschliesslich um mehrphasige feste Lösungen handelt, d.h. die entsprechenden Röntgenbeugungsdiagramme weisen ausser den neuen Linien der festen Lösung auch noch Linien der einen und/oder der anderen Einzelkomponente auf.

Es ist nun gefunden worden, dass entsprechend behandelte Gemische zweier unterschiedlicher symmetrischer 1,4-Diketopyrrolopyrrole der Typen 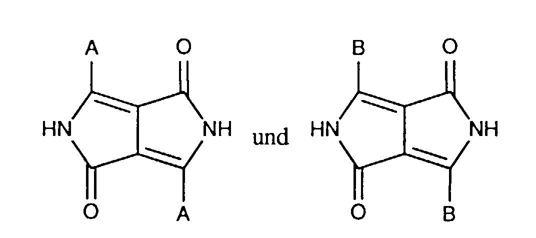 im Molverhältnis 1:1, neue Mischkristalle bilden, die ganz überraschend mit den Kristallen der entsprechenden asymmetrischen 1,4-Diketopyrrolopyrrole des Typs 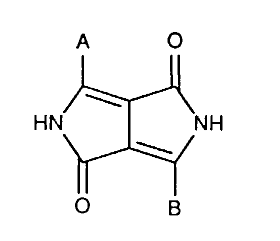 isomorph sind. Es handelt sich dabei um einphasige Produkte, deren Röntgenbeugungsspektrum sich sowohl von denjenigen der Einzelkomponenten des Mischkristalls als auch von demjenigen ihres physikalischen Gemisches unterscheidet. Das Röntgenbeugungsspektrum des Mischkristalls und dasjenige der asymmetrischen Einzelverbindung sind hingegen identisch.

Die vorliegende Erfindung betrifft demnach Mischkristalle von 1,4-Diketopyrrolo[3,4-c]-pyrrolen, bestehend aus zwei unterschiedlichen Verbindungen der Formeln 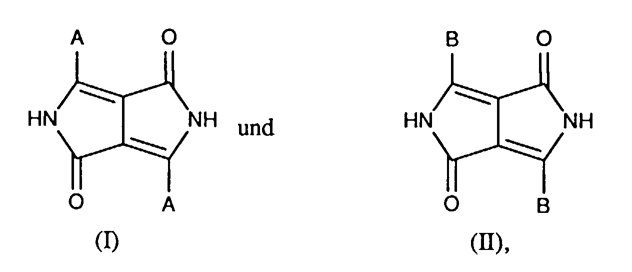 im Molverhältnis 1:1, worin A und B, die verschieden sein müssen, jeweils für eine Gruppe der Formel 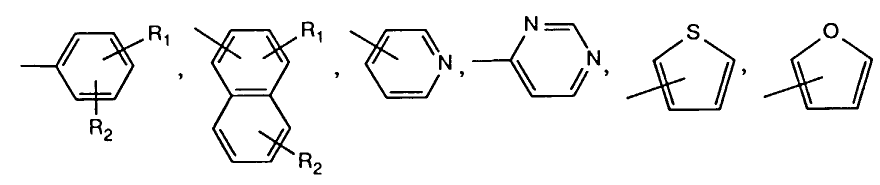 oder 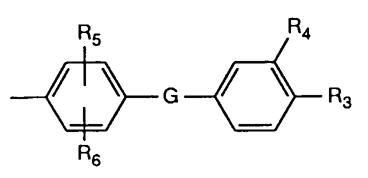 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₁-C₁₈-Alkoxycarbonyl, C₁-C₁₈-Alkylaminocarbonyl, -CN, -NO₂, Trifluormethyl, C₅-C₆-Cycloalkyl, -C=N-(C₁-C₁₈-Alkyl), 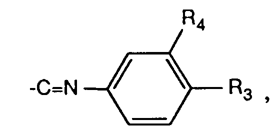 Imidazolyl, Pyrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂-, -CONH- oder -NR₇- ist,
R₃ und R₄ unabhängig voneinander Wasserstoff, Halogen, C₁-C₆-Alkyl, C₁-C₁₈Alkoxy oder -CN sind, R₅ und R₆ unabhängig voneinander Wasserstoff, Halogen oder C₁-C₆-Alkyl und R₇ Wasserstoff oder C₁-C₆-Alkyl bedeuten.

Bedeuten etwaige Substitutenten Halogen, dann handelt es sich z.B. um Jod, Fluor, insbesondere Brom und bevorzugt Chlor;
bei C₁-C₆-Alkyl handelt es sich beispielsweise um Methyl, Ethyl, n-Propyl, Isopropyl, n-Butyl, sec.-Butyl, tert.-Butyl, n-Amyl, tert.-Amyl, Hexyl und bei C₁-C₁₈-Alkyl zusätzlich z.B. um Heptyl, Octyl, 2-Ethylhexyl, Nonyl, Decyl, Dodecyl, Tetradecyl, Hexadecyl oder Octadecyl;
C₁-C₁₈-Alkoxy bedeutet, auch in C₁-C₁₈-Alkoxycarbonyl, z.B. Methoxy, Ethoxy, n-Propoxy, Isopropoxy, Butyloxy, Hexyloxy, Decyloxy, Dodecyloxy, Hexadecyloxy oder Octadecyloxy;
C₁-C₁₈-Alkylmercapto steht beispielsweise für Methylmercapto, Ethylmercapto, Propylmercapto, Butylmercapto, Octylmercapto, Decylmercapto, Hexadecylmercapto oder Octadecylmercapto;
C₁-C₁₈-Alylamino bedeutet, auch in C₁-C₁₈-Alkylaminocarbonyl, z.B. Methylamino, Ethylamino, Propylamino, Hexylamino, Decylamino, Hexadecylamino oder Octadecylamino.
C₅-C₆-Cycloalkyl steht z.B. für Cyclopentyl und insbesondere für Cyclohexyl.

Von besonderem Interesse sind die erfindungsgemässen Mischkristalle bei denen in den Formeln I und II A und B jeweils eine Gruppe der Formel 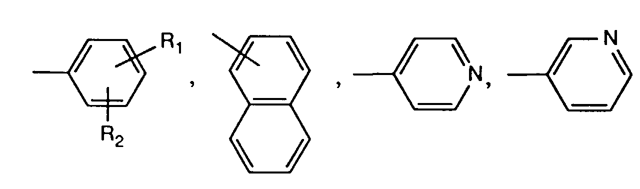 oder 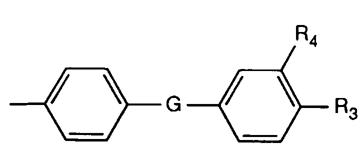 sind,
worin
R₁ und R₂ unabhängig voneinander Wasserstoff, Chlor, Brom, C₁-C₄-Alkyl, C₁-C₆-Alkoxy, C₁-C₆-Alkylamino oder CN bedeuten,
   G -O-, -NR₇-, -N=N- oder -SO₂- ist,
R₃ und R₄ Wasserstoff und R₇ Wasserstoff, Methyl oder Ethyl bedeuten,
und insbesondere jene, worin in den Formeln I und II A und B jeweils eine Gruppe der Formel 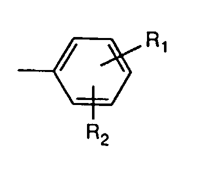 sind, worin R₁ und R₂ unabhängig voneinander Wasserstoff, Methyl, tert.-Butyl, Chlor, Brom oder CN bedeuten. R₂ ist bevorzugt Wasserstoff.

Die erfindungsgemässen Mischkristalle können ausgehend von physikalischen Mischungen der oben definierten Komponenten der Formeln I und II nach folgenden an und für sich bekannten Verfahren hergestellt werden:
- durch Kontaktierung in polaren organischen Lösungsmitteln, bevorzugt durch Verrühren der Komponentenmischung bei Rückflusstemperatur,
- durch alkalische Umfällung der Komponentenmischung in polaren organischen Lösungsmitteln oder durch Verrühren der Komponentenmischung in polaren organischen Lösungsmitteln in Gegenwart von Alkalialkoholaten, Alkalihydroxiden oder quaternären Ammoniumverbindungen oder
- durch saure Umfällung, d.h. Auflösung der Komponentenmischung in Säure und Fällung der festen Lösung durch Verdünnen mit Wasser,
wobei in Analogie zu den Verfahren, wie sie z.B. in US-Patent 4 783 540 detailliert beschrieben sind, vorgegangen werden kann.

Eine neue Herstellungsmethode besteht darin, dass die Verbindungen der Formeln I und II nach an sich bekannten Methoden mit einem Dicarbonat der Formel

D - O - D (III)

oder mit einem Trihaloessigsäureester der Formel

(R₈)₃C - D (IV),

oder mit einem Azid der Formel

DN₃ (V),

oder mit einem Carbonat der Formel

D-OR₉ (VI),

oder mit einem Alkylideniminooxyameisensäureester der Formel 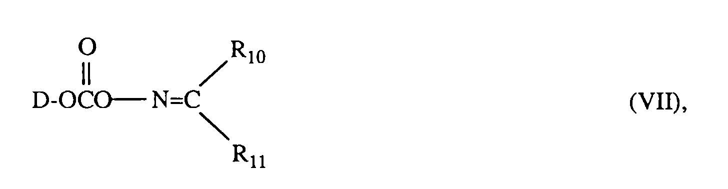 worin D eine Gruppe der Formel 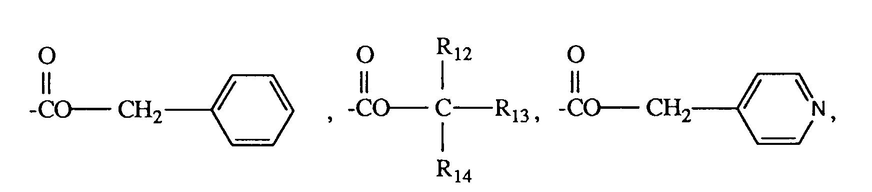 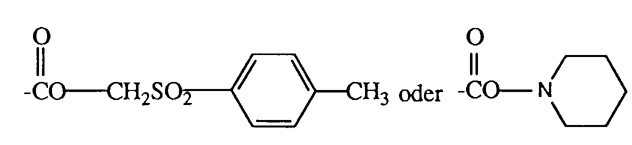 bedeutet, R₈ Chlor, Fluor oder Brom, R₉ C₁-C₄-Alkyl oder unsubstituiertes oder durch Halogen, C₁-C₄-Alkyl, C₁-C₄-Alkoxy oder -CN substituiertes Phenyl, R₁₀ -CN oder -COOR₉ und R₁₁ unsubstituiertes oder durch Halogen, C₁-C₄-Alkyl, C₁-C₄-Alkoxy oder -CN substituiertes Phenyl sind und R₁₂, R₁₃ und R₁₄ unabhängig voneinander Wasserstoff, C₁-C₆-Alkyl oder C₂-C₅-Alkenyl bedeuten, wobei mindestens zwei von R₁₂, R₁₃ und R₁₄ Alkyl oder Alkenyl sein müssen, im Molverhältnis 1:2 in einem aprotischen organischen Lösungsmittel in Gegenwart einer Base als Katalysator, in lösliche Verbindungen der Formeln 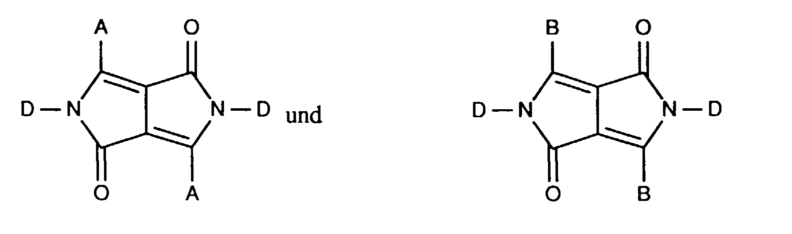 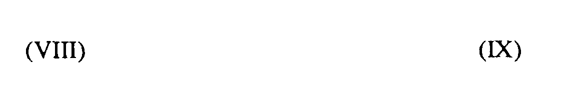 umgesetzt werden,
- diese Verbindungen entweder
   a) nach allgemein bekannten Methoden in Pulverform im Molverhältnis 1:1 homogen vermischt werden oder
   b) nach allgemein bekannten Methoden in Pulverform im Molverhältnis 1:1 homogen vermischt werden und das Gemisch in einem Lösungsmittel gelöst wird oder
   c) zuerst gelöst werden und in Lösung im Mischverhältnis 1:1 vermischt werden
   und danach
- aus dem trockenen oder gelösten Gemisch durch thermische, photolytische oder chemische Behandlung der gewünschte Mischkristall ausgefällt wird.

R₁₂, R₁₃ und R₁₄ bedeuten als C₂-C₅Alkenyl z.B. Vinyl, Allyl, Methallyl, n-But-2-enyl, 2-Methyl-prop-2-enyl oder n-Pent-2-enyl.

Bevorzugt sind R₁₂ und R₁₄ Methyl und R₁₃ C₁-C₆-Alkyl und insbesondere Methyl.

Bevorzugt ist D eine Gruppe der Formel

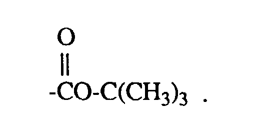

Bevorzugt werden die Verbindungen der Formeln I und II mit einem Dicarbonat der Formel III umgesetzt.

Die Dicarbonate der Formel III, Trihaloessigsäureester der Formel IV, Azide der Formel V, Carbonate der Formel VI und Alkylideniminooxyameisensäureester der Formel VII sind bekannte Substanzen. Sollten einige noch neu sein, so können sie in Analogie zu allgemein bekannten Methoden hergestellt werden.

Geeignete aprotische organische Lösungsmittel sind beispielsweise Ether, wie Tetrahydrofuran oder Dioxan, oder Glykoläther, wie Ethylenglykol-methyläther, Ethylenglykolethylether, Diethylenglykol-monomethylether oder Diethylenglykol-monoethylether, ferner dipolar-aprotische Lösungsmittel, wie Acetonitril, Benzonitril, N,N-Dimethylformamid, N,N-Dimethylacetamid, Nitrobenzol, N-Methylpyrrolidon, halogenierte aliphatische oder aromatische Kohlenwasserstoffe, wie Trichlorethan, Benzol oder durch Alkyl, Alkoxy oder Halogen substituiertes Benzol, wie Toluol, Xylol, Anisol oder Chlorbenzol oder aromatische N-Heterocyclen, wie Pyridin, Picolin oder Chinolin. Bevorzugte Lösungsmittel sind z.B. Tetrahydrofuran, N,N-Dimethylformamid, N-Methylpyrrolidon. Die genannten Lösungsmittel können auch als Mischungen eingesetzt werden. Zweckmässigerweise verwendet man 5-20 Gew.-Teile Lösungsmittel auf 1 Gew.-Teil der Reaktionsteilnehmer.

Als Katalysator geeignete Basen sind beispielsweise die Alkalimetalle selbst, wie Lithium-, Natrium- oder Kalium sowie deren Hydroxide und Carbonate, oder Alkaliamide, wie Lithium-, Natrium- oder Kaliumamid oder Alkalihydride, wie Lithium-, Natrium- oder Kaliumhydrid, oder Erdalkali- oder Alkalialkoholate, die sich insbesondere von primären, sekundären oder tertiären aliphatischen Alkoholen mit 1 bis 10 C-Atomen ableiten, wie z.B. Lithium-, Natrium- oder Kaliummethylat, -ethylat, -n-propylat, -isopropylat, n-butylat, -sek.-butylat, tert.-butylat, -2-methyl-2-butylat, -2-methyl-2-pentylat, -3-methyl-3-pentylat, -3-ethyl-3-pentylat, und ferner organische aliphatische, aromatische oder heterocyclische N-Basen, darunter z.B. Diazabicycloocten, Diazabicycloundecen und 4-Dimethylaminopyridin und Trialkylamine, wie z.B. Trimethyl- oder Triethylamin. Man kann aber auch ein Gemisch der genannten Basen verwenden.

Bevorzugt werden die organischen N-Basen, wie z.B. Diazabicycloocten, Diazabicycloundecen und insbesondere 4-Dimethylaminopyridin.

Die Umsetzung wird zweckmässig bei Temperaturen zwischen 10 und 100°C, insbesondere zwischen 14 und 40°C, durchgeführt und zwar bei atomsphärischem Druck.

Die Verbindungen der Formel I oder II werden entweder nach allgemein bekannten Methoden in Pulverform im gewünschten Verhältnis vermischt und das Gemisch im Lösungsmittel gelöst oder sie werden zuerst gelöst und die Lösungen im gewünschten Verhältnis vermischt.

Es können zweckmässig folgende Lösungsmittel eingesetzt werden: Ether, wie Tetrahydrofuran oder Dioxan, oder Glykoläther, wie Ethylenglykol-methyläther, Ethylenglykol-ethylether, Diethylenglykol-monomethylether oder Diethylenglykol-monoethylether, Polyalkohole, wie Polyethylenglykol, Ketone, wie Aceton, Ethylmethylketon, Isobutylmethylketon oder Cyclohexanon, ferner dipolar-aprotische Lösungsmittel, wie Acetonitril, Benzonitril, N,N-Dimethylformamid, N,N-Dimethylacetamid, Nitrobenzol, N-Methylpyrrolidon, Dimethylsulfoxyd, halogenierte aliphatische oder aromatische Kohlenwasserstoffe, wie Trichlorethan, Dichlormethan, Chloroform, Benzol oder durch Alkyl, Alkoxy oder Halogen substituiertes Benzol, wie Toluol, Xylol, Anisol oder Chlorbenzol, aromatische N-Heterocyclen, wie Pyridin, Picolin oder Chinolin oder hochsiedende Lösungsmittel, wie Decalin, n-Dodecan oder Kerosin oder Gemische derselben. Bevorzugte Lösungsmittel sind z.B. Toluol, Diphenylether, N-Methylpyrrolidon, N,N-Dimethylformamid, Dimethylsulfoxyd und Chinolin.

Die Konzentration der Verbindungen der Formel I bzw. II im Lösungsmittel oder Lösungsmittelsystem kann je nach Lösungsmittel stark variieren. Zweckmässig setzt man 0,1 bis 20 Gew.% Verbindung der Formel I bzw. II, bezogen auf die gesamte Lösung und vorzugsweise 0,2 bis 5 Gew.% ein.

Aus dem trockenen oder gelösten Gemisch der Verbindungen der Formeln VIII und IX können die Mischkristalle bestehend aus den Verbindungen der Formeln I und II auf einfachste Weise erhalten werden, sei es durch a) thermische, d.h. z.B. durch Aufheizen auf Temperaturen zwischen 50 und 400°C, bevorzugt zwischen 100 und 200°C oder Laserbestrahlung, b) photolytische, d.h. z.B. durch Belichtung mit Wellenlängen unter 375 nm, oder c) chemische, d.h. z.B. mit organischen oder anorganischen Säuren, wie beispielsweise Essig-, Toluolsulfon-, Trifluoressig-, Salz- oder Schwefelsäure, Behandlung des trockenen oder gelösten Gemisches und Isolierung des erhaltenen Produktes nach üblichen Methoden.

Das Röntgenbeugungsdiagramm der erfindungsgemässen Mischkristalle ist wie bereits erwähnt durch andere Linien gekennzeichnet als diejenigen, welche die Röntgenbeugungsdiagramme des entsprechenden physikalischen Gemischs und der entsprechenden Einzelkomponenten charakterisieren, deckt sich aber weitgehend mit demjenigen des asymmetrischen Diketopyrrolopyrrols des Typs

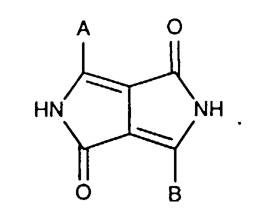

Es ist aber auch gefunden worden, dass beim Einsetzen eines kleinen Ueberschusses des die kleinere geometrische Konstitution aufweisenden 1,4-Diketopyrrolopyrrols, entgegen den Erwartungen, wonach der grössere Anteil das Kristallgitter bilden sollte (Wirtsgitter), in welchem sich der kleinere Anteil als Gast einlagert, überraschenderweise zuerst das obenbeschriebene 1:1-molare Mischkristall entsteht, in dessen Gitter sich der überschüssige Anteil unter Bildung einer festen Lösung einlagert. Die erhaltene einphasige feste Lösung besitzt demnach dasselbe Kristallgitter wie das 1:1-molare Mischkristall und die entsprechenden Röntgenbeugungsdiagramme sind praktisch identisch.

Unter Diketopyrrolopyrrolen, die eine kleinere geometrische Konstitution aufweisen, versteht man Verbindungen mit kleineren Molekulardimensionen (kleinere sterische Hinderung), d.h. mit der kleineren Raumbeanspruchung. Bezogen auf die Bedeutungen von A und B ist z.B
unsubstituiertes Phenyl < substituiertes Phenyl;
p-Methylphenyl < p-tert.-Butylphenyl;
Cyanphenyl < Chlorphenyl; usw.

Durch die Bildung solcher festen Lösungen können sehr interessante und vorteilhafte Nuancenverschiebungen erzielt werden, ohne die guten Pigmenteigenschaften zu beeinträchtigen.

Ein weiterer Gegenstand der vorliegenden Erfindung sind demzufolge einphasige feste Lösungen von 1,4-Diketopyrrolo[3,4-c]pyrrolen, bestehend aus zwei unterschiedlichen Verbindungen der Formeln 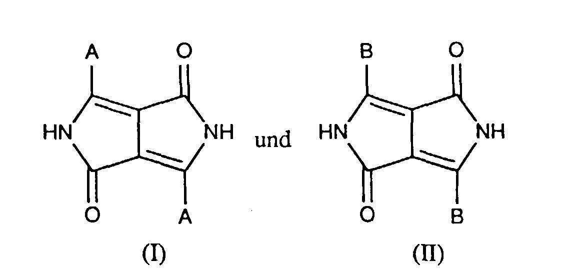 mit der oben angegebenen Bedeutung,
mit der Massgabe, dass das die kleinere geometrische Konstitution aufweisende 1,4-Diketopyrrolo[3,4-c]pyrrol in einer Menge von 50 bis 70 Mol%, bevorzugt 55 bis 60 Mol%, enthalten ist.

Für die Substituenten A und B gelten auch hier die oben bereits für die 1:1-molaren Mischkristalle angegebenen Bevorzugungen.

Die Herstellung der erfindungsgemässen festen Lösungen erfolgt, mit Ausnahme der einzusetzenden Menge der beiden Komponenten, nach den genau gleichen Methoden, wie für die erfindungsgemässen 1:1-molaren Mischkristalle.

Die Rekristallisation bzw. thermische Behandlung geschieht nach für Pigmente üblichen Methoden. Im allgemeinen handelt es sich um eine thermische Nachbehandlung in Wasser oder in einem organischen Lösungsmittel, gegebenenfalls unter Druck. Vorzugsweise verwendet man organische Lösungsmittel, z.B. durch Halogenatome, Alkyl- oder Nitrogruppen substituierte Benzole, wie Xylole, Chlorbenzol, o-Dichlorbenzol oder Nitrobenzol, sowie Pyridinbasen, wie Pyridin, Picolin oder Chinolin, ferner Ketone, wie Cyclohexanon, Alkohole, wie Isopropanol, Butanole oder Pentanole, Ether, wie Ethylenglykolmonomethyl- oder -monoethylether, Amide, wie Dimethylformamid oder N-Methylpyrrolidon, sowie Dimethylsulfoxid oder Sulfolan. Man kann die Nachbehandlung auch in Wasser, gegebenenfalls unter Druck, in Gegenwart von organischen Lösungsmitteln und/oder mit Zusatz von oberflächenaktiven Substanzen durchführen.

Sowohl die erfindungsgemässen Mischkristalle als auch die erfindungsgemässen festen Lösungen können als Pigmente zum Färben von hochmolekularem organischem Material verwendet werden.

Hochmolekulare organische Materialien, die mit den erfindungsgemässen Mischkristallen oder festen Lösungen gefärbt bzw. pigmentiert werden können, sind z.B. Celluloseether und -ester, wie Ethylcellulose, Nitrocellulose, Celluloseacetat, Cellulosebutyrat, natürliche Harze oder Kunstharze, wie Polymerisationsharze oder Kondensationsharze, wie Aminoplaste, insbesondere Harnstoff- und Melamin-Formaldehydharze, Alkydharze, Phenoplaste, Polycarbonate, Polyolefine, Polystyrol, Polyvinylchlorid, Polyamide, Polyurethane, Polyester, ABS, Polyphenylenoxide, Gummi, Casein, Silikon und Silikonharze, einzeln oder in Mischungen.

Die erwähnten hochmolekularen organischen Verbindungen können einzeln oder in Gemischen als plastische Massen, Schmelzen oder in Form von Spinnlösungen, Lacken, Anstrichstoffen oder Druckfarben vorliegen. Je nach Verwendungszweck erweist es sich als vorteilhaft, die erfindungsgemässen Mischkristalle oder festen Lösungen als Toner oder in Form von Präparaten einzusetzen.

Bezogen auf das zu pigmentierende hochmolekulare organische Material kann man die erfindungsgemässen Mischkristalle oder festen Lösungen in einer Menge von 0,01 bis 30 Gew.%, vorzugsweise von 0,1 bis 10 Gew. %, einsetzen.

Die Pigmentierung der hochmolekularen organischen Substanzen mit den erfindungsgemässen Mischkristallen oder festen Lösungen erfolgt beispielsweise derart, dass man solche Mischkristalle bzw. festen Lösungen gegebenenfalls in Form von Masterbatches diesen Substraten unter Verwendung von Walzwerken, Misch- oder Mahlapparaten zumischt. Das pigmentierte Material wird hierauf nach an sich bekannten Verfahren, wie Kalandrieren, Pressen, Strangpressen, Streichen, Giessen oder Spritzgiessen, in die gewünschte endgültige Form gebracht. Oft ist es erwünscht, zur Herstellung von nicht starren Formlingen oder zur Verringerung ihrer Sprödigkeit den hochmolekularen Verbindungen vor der Verformung sogenannte Weichmacher einzuverleiben. Als solche können z.B. Ester der Phosphorsäure, Phthalsäure oder Sebacinsäure dienen. Die Weichmacher können vor oder nach der Einverleibung der erfindungsgemässen Mischkristalle bzw. festen Lösungen in die Polymeren eingearbeitet werden. Zwecks Erzielung verschiedener Farbtöne ist es ferner möglich, den hochmolekularen organischen Stoffen neben den erfindungsgemässen Mischkristallen bzw. festen Lösungen noch Füllstoffe bzw. andere farbgebende Bestandteile, wie Weiss-, Bunt- oder Schwarzpigmente, in beliebigen Mengen zuzufügen.

Zum Pigmentieren von Lacken, Anstrichstoffen und Druckfarben werden die hochmolekularen organischen Materialien und die erfindungsgemässen Mischkristalle oder festen Lösungen gegebenenfalls zusammen mit Zusatzstoffen, wie Füllmitteln, anderen Pigmenten, Siccativen oder Weichmachern, in einem gemeinsamen organischen Lösungsmittel oder Lösungsmittelgemisch fein dispergiert bzw. gelöst. Man kann dabei so verfahren, dass man die einzelnen Komponenten für sich oder auch mehrere gemeinsam dispergiert bzw. löst, und erst hierauf alle Komponenten zusammenbringt.

Besonders geeignet sind die erfindungsgemässen Mischkristalle und festen Lösungen zum Einfärben von Kunststoffen, insbesondere Polyvinylchlorid und Polyolefinen, und Lacken, insbesondere Automobillacken.

In Färbungen, beispielsweise von Polyvinylchlorid oder Polyolefinen, zeichnen sich sowohl die erfindungsgemässen Mischkristalle als auch die erfindungsgemässen festen Lösungen durch gute allgemeine Pigmenteigenschaften, wie gute Dispergierbarkeit, hohe Farbstärke und Reinheit, gute Migrations-, Hitze-, Licht- und Wetterbeständigkeit sowie gute Deckkraft aus.

Die nachfolgenden Beispiele erläutern die Erfindung.

Beispiel 1: a) Einer Mischung aus 14,75 g (0,0512 Mol) 1,4-Diketo-3,6-diphenyl-pyrrolo-[3,4-c]pyrrol und 3,23 g (0,0264 Mol) 4-Dimethylaminopyridin in 500 ml Tetrahydrofuran (über Molekularsieb getrocknet) werden in 3 Portionen im Abstand von einer Stunde 27,94 g (0,128 Mol) Di-tert.-butyl-dicarbonat zugegeben. Die erhaltene rote Suspension wird 2 Stunden bei Raumtemperatur unter Ausschluss von atmosphärischer Feuchtigkeit gerührt. Man erhält eine dunkelgrüne Lösung. Das Lösungsmittel wird bei vermindertem Druck abdestilliert. Der gelbe Rückstand wird mit einer 5 %igen wässrigen Natriumbicarbonatlösung gewaschen, mit Wasser ausgespült und im Vakuum bei Raumtemperatur getrocknet. Man erhält 24,5 g (98 % d.Th.) N,N-Di-tert.-butoxycarbonyl-1,4-diketo-3,6-diphenylpyrrolo[3,4-c]pyrrol

### Analyse:

¹H-NMR (CDCl₃): 7,75 (d, 4H); 7,48-7,50 (m, 6H); 1,40 (s, 18H).

b) Einer Mischung aus 8,44 g (0,021 Mol) 1,4-Diketo-3,6-di-(4-tert.-butylphenyl)-pyrrolo-[3,4-c]pyrrol und 1,49 g (0,012 Mol) 4-Dimethylaminopyridin in 100 ml N,N-Dimethylformamid (über Molekularsieb getrocknet) werden 24,29 g (0,111 Mol) Di-tert.-butyl-dicarbonat zugegeben. Die erhaltene rote Suspension wird 3 Stunden bei Raumtemperatur unter Ausschluss von atmosphärischer Feuchtigkeit gerührt. Es erfolgt ein Farbumschlag nach Orange. Die ausgefallene Substanz wird abfiltriert, der Rückstand wird wiederholt mit kaltem destilliertem Wasser gewaschen und im Vakuum bei Raumtemperatur getrocknet. Man erhält 11,40 g (90 % d.Th.) N,N-Di-tert.-butoxycarbonyl-1,4-diketo-3,6-di-(4-tert.-butylphenyl)-pyrrolo[3,4-c]pyrrol als leuchtend gelbes Produkt.

### Analyse:

¹H-NMR (CDCl₃): 7,69 (d, 4H); 7,48 (d, 4H); 1,43 (s, 18H); 1,34 (s, 18H).

c) Eine Mischung von 1,50 g (3,07 mMol) N,N'-Di-tert.-butoxycarbonyl-1,4-diketo-3,6-diphenyl-pyrrolo[3,4-c]pyrrol (a) und 1,84 g (3,07 mMol) N,N'-Di-tert.-butoxycarbonyl-1,4-diketo-3,6-di-(4-tert.-butylphenyl)-pyrrolo[3,4-c]pyrrol (b) wird in 100 ml Toluol bei Raumtemperatur gelöst. Die gelbe Lösung wird unter Rühren auf 70°C erhitzt, dann werden 2,90 g Toluol-4-sulfonsäure-monohydrat zugegeben, auf 100°C erhitzt, bei dieser Temperatur 16 Std. gerührt und anschliessend auf Raumtemperatur abkühlen gelassen. Die gebildete purpurrote feste Substanz wird abfiltriert, zuerst mit Methanol, dann mit destilliertem Wasser gewaschen und im Vakuumtrockenschrank bei 60°C getrocknet. Man erhält 1,86 g (87,9 % d.Th) eines purpurroten Pulvers.

| Analyse | C | H | N |
|---|---|---|---|
| Ber. | 76,73 % | 5,85 % | 8,13 % |
| Gef. | 76,80 % | 5,82 % | 8,05 % |

Die vollständigen Röntgenbeugungsdiagramme werden nach üblichen Methoden mit Hilfe eines Siemens D 500® Röntgen-Diffraktometers (CuK_{α}-Strahlung) bestimmt.

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 18,9113 | 4,669 | 100 |
| 6,1915 | 14,294 | 7 |
| 4,9491 | 17,908 | 43 |
| 3,3535 | 26,559 | 48 |
| 3,2997 | 26,559 | 22 |

gekennzeichnet.

Zum Vergleich ist das Röntgenbeugungsdiagramm des bekannten asymmetrischen Diketopyrrolopyrrols der Formel 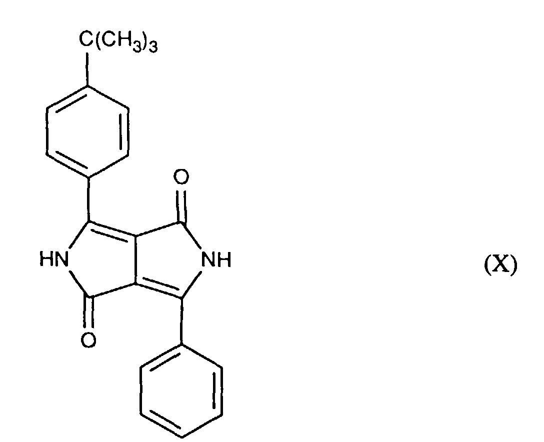 durch folgende Beugungslinien

| Netzebenenabstände (d-Werte in Å) | doppelte Glanzwinkel (2Θ) | relative Intensität |
|---|---|---|
| 18,5598 | 4,757 | 100 |
| 6,1761 | 14,329 | 8 |
| 4,9262 | 17,992 | 27 |
| 3,3446 | 26,631 | 32 |
| 3,2901 | 27,080 | 15 |

gekennzeichnet.

Der Mischkomplex verhält sich in PVC- und Lackfärbungen identisch wie das entsprechende asymmetrische Diketopyrrolopyrrol der Formel X.

Beispiel 2: a) Einer Suspension von 20,0 g 1,4-Diketo-3,6-di-(4-chlorphenyl)-pyrrolo[3,4-c]pyrrol in 500 ml N,N'-Dimethylformamid werden 1,78 g 4-Dimethylaminopyridin und danach 26,8 g Di-tert.-butyl-dicarbonat zugegeben. Das Reaktionsgemisch wird bei Raumtemperatur unter Ausschluss von atmosphärischer Feuchtigkeit gerührt. Nach 15 Stunden werden erneut 26,8 g Di-tert.-butyl-dicarbonat zugegeben und 30 Stunden weitergerührt. Das ausgefallene braun-orange Produkt wird abfiltriert, mit Methanol gewaschen und im Vakuum bei Raumtemperatur getrocknet. Man erhält 21,8 g (70 % d.Th.) des Produktes der Formel

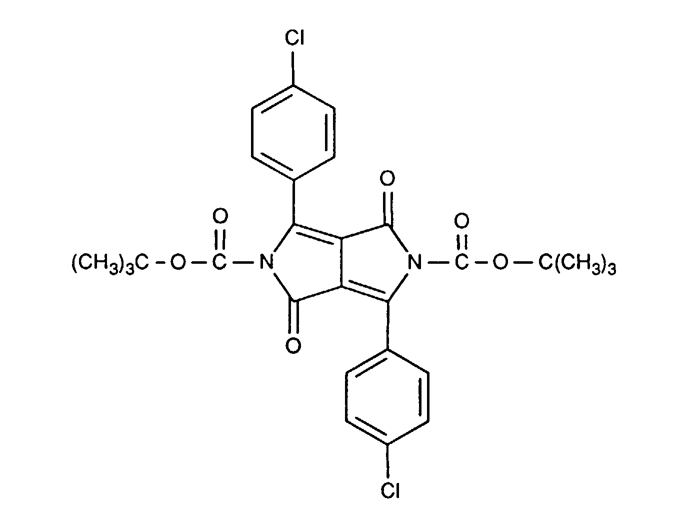

| Analyse | C | H | N | Cl |
|---|---|---|---|---|
| Ber. | 60,33 % | 4,70 % | 5,03 % | 12,72 % |
| Gef. | 60,24 % | 4,79 % | 4,92 % | 12,50 % |

b) Einer Mischung von 10,0 g 1,4-Diketo-3,6-di-(3-methylphenyl)-pyrrolo[3,4-c]pyrrol und 1,0 g 4-Dimethylaminopyridin in 350 ml Tetrahydrofuran werden 15,2 g Di-tert.-butyldicarbonat zugegeben. Die erhaltene orange Suspension wird 20 Stunden bei Raumtemperatur unter Ausschluss von atmosphärischer Feuchtigkeit gerührt. Danach wird das Lösungsmittel bei vermindertem Druck abdestilliert. Der braune Rückstand wird zuerst mit Wasser, dann mit Methanol gewaschen und im Vakuum bei Raumtemperatur getrocknet. Man erhält 14,1 g (86,5 % d.Th.) eines leuchtend gelben Produktes der Formel

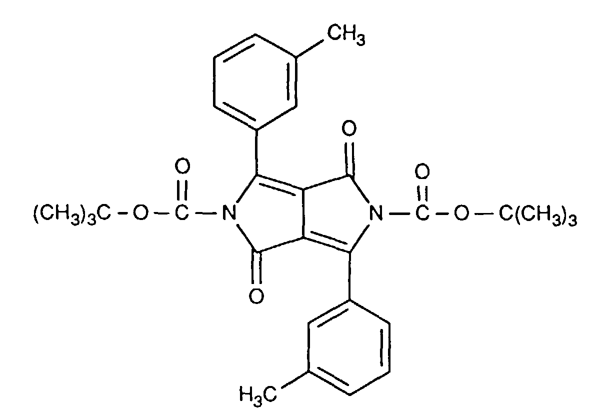

| Analyse | C | H | N |
|---|---|---|---|
| Ber. | 69,75 % | 6,24 % | 5,42 % |
| Gef. | 69,82 % | 6,40 % | 5,47 % |

c) Eine Mischung von 6,97 g (12,5 mMol) des Produktes aus a) und 6,46 g (12,5 mMol) des Produktes aus b) in 400 ml Toluol wird unter Rühren auf 60°C erhitzt dann werden 11,89 g Toluol-4-sulfonsäure zugegeben, auf 100°C geheizt, bei dieser Temperatur 2 Stunden gerührt und anschliessend auf Raumtemperatur abkühlen gelassen. Das ausgefallene Pigment wird abfiltriert, in 300 ml Methanol auf 60°C erhitzt und 30 Minuten bei dieser Temperatur gerührt. Danach wird das Produkt abfiltriert, zuerst mit Methanol, dann mit destilliertem Wasser gewaschen und im Vakuum bei 60°C getrocknet. Man erhält 6,8 g (81 % d.Th.) eines roten Pulvers.

| Analyse | C | H | N | Cl |
|---|---|---|---|---|
| Ber. | 67,76 % | 3,89 % | 8,32 % | 10,53 % |
| Gef. | 66,92 % | 3,89 % | 8,24 % | 11,13 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 15,3172 | 5,765 | 99 |
| 7,5658 | 11,687 | 14 |
| 6,8504 | 12,913 | 26 |
| 6,3196 | 14,003 | 34 |
| 6,1515 | 14,387 | 48 |
| 5,0223 | 17,645 | 22 |
| 3,6887 | 24,107 | 22 |
| 3,3206 | 26,827 | 100 |
| 3,1567 | 28,248 | 17 |

gekennzeichnet.

Zum Vergleich ist das Röntgenbeugungsdiagramm des bekannten asymmetrischen Diketopyrrolopyrrols der Formel 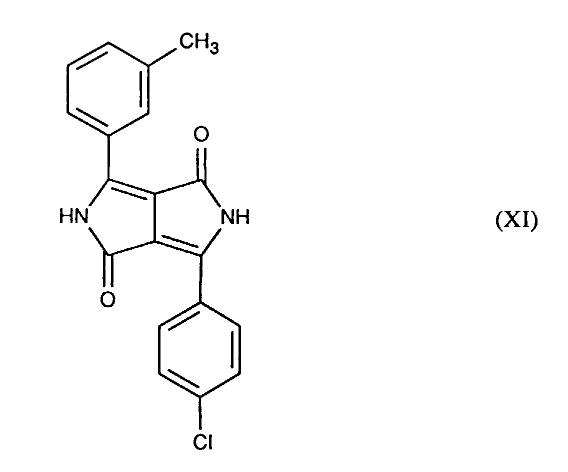 durch folgende Beugungslinien

| Netzebenenabstände (d-Werte in Å) | doppelte Glanzwinkel (2Θ) | relative Intensität |
|---|---|---|
| 15,3005 | 5,772 | 100 |
| 7,5601 | 11,696 | 13 |
| 6,8195 | 12,971 | 24 |
| 6,2099 | 14,251 | 54 |
| 5,0456 | 17,563 | 24 |
| 3,6945 | 24,069 | 22 |
| 3,3298 | 26,751 | 80 |
| 3,1446 | 28,359 | 17 |

gekennzeichnet.

Der Mischkomplex verhält sich in PVC- und Lackfärbungen identisch wie das entsprechende asymmetrische Diketopyrrolopyrrol der Formel XI.

### Beispiel 3: a) (Herstellung des Mischkristalls)

Eine Mischung von 7,21 g (12,0 mMol) des Produktes aus Beispiel 1b) und 6,69 g (12,0 mMol) des Produktes aus Beispiel 2a) wird in 380 ml Toluol unter Rühren auf 60°C erhitzt. Anschliessend werden 11,41 g Toluol-4-sulfonsäure zugegeben. Man erwärmt auf 100°C, rührt 2 Stunden bei dieser Temperatur und kühlt anschliessend auf Raumtemperatur ab. Das ausgefallene Pigment wird abfiltriert, zuerst mit Methanol, dann mit destilliertem Wasser gewaschen und im Vakuum bei 80°C getrocknet. Man erhält 8,27 g (91 % d.Th) eines roten Pulvers.

| Analyse | C | H | N | Cl |
|---|---|---|---|---|
| Ber. | 69,25 % | 4,94 % | 7,42 % | 9,93 % |
| Gef. | 69,67 % | 5,05 % | 7,40 % | 9,32 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 19,4208 | 4,55 | 59 |
| 6,3517 | 13,93 | 15 |
| 4,9880 | 17,77 | 83 |
| 3,7947 | 23,42 | 7 |
| 3,6444 | 24,40 | 7 |
| 3,3649 | 26,47 | 100 |
| 3,2301 | 27,59 | 31 |
| 3,1587 | 28,23 | 8 |
| 3,0305 | 29,45 | 9 |

gekennzeichnet.

### b) (Herstellung der festen Lösung aus den löslichen Diketopyrrolopyrrolen)

Eine Mischung von 2,40 g (4,0 mMol) des Produktes aus Beispiel 1b) und 3,34 g (6,0 mMol) des Produktes aus Beispiel 2a) wird in 150 ml Toluol unter Rühren auf 60°C erhitzt. Zur entstandenen Lösung werden 2,38 g Toluol-4-sulfonsäure-monohydrat zugegeben und auf 100°C erhitzt. Diese Mischung wird bei dieser Temperatur 2 Stunden gerührt. Anschliessend lässt man auf Raumtemperatur abkühlen. Die gebildete rote feste Substanz wird abfiltriert, zuerst mit Methanol, dann mit Wasser gewaschen und im Vakuumtrockenschrank bei 80°C getrocknet. Man erhält 3,5 g (93 % d.Th) eines roten Pulvers.

| Analyse | C | H | N | Cl |
|---|---|---|---|---|
| Ber. | 67,99 % | 4,63 % | 7,48 % | 11,36 % |
| Gef. | 67,93 % | 4,65 % | 7,52 % | 11,48 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 19,3152 | 4,57 | 70 |
| 6,3410 | 13,96 | 19 |
| 4,9820 | 17,79 | 89 |
| 3,7899 | 23,45 | 9 |
| 3,6375 | 24,45 | 11 |
| 3,3617 | 26,49 | 100 |
| 3,2272 | 27,62 | 33 |
| 3,1664 | 28,16 | 16 |
| 3,0262 | 29,49 | 11 |

gekennzeichnet.

Daraus erkennt man, dass die Kristallstruktur dieser festen Lösung mit derjenigen des entsprechenden Mischkristalls (a) praktisch identisch ist.

### Beispiel 4 a) (Herstellung des löslichen Diketopyrrolopyrrols)

Einer Mischung von 10,15 g 1,4-Diketo-3,6-di-(4-cyanphenyl)-pyrrolo[3,4-c]pyrrol und 19,6 g Di-tert.-butyl-dicarbonat in 400 ml Tetrahydrofuran werden 0,92 g 4-Dimethylaminopyridin zugegeben. Die erhaltene rote Suspension wird 20 Stunden bei Raumtemperatur unter Ausschluss von atmosphärischer Feuchtigkeit gerührt. Das Lösungsmittel wird bei vermindertem Druck abdestilliert. Der braune Rückstand wird mit Methanol gewaschen und im Vakuum bei Raumtemperatur getrocknet. Man erhält 10,6 g (66 % d.Th.) N,N'-Di-tert.-butoxycarbonyl- 1,4-diketo-3,6-di-(4-cyanphenyl)-pyrrolo[3,4-c]pyrrol.

| Analyse | C | H | N |
|---|---|---|---|
| Ber. | 66,91 % | 4,87 % | 10,40 % |
| Gef. | 66,84 % | 5,02 % | 10,32 % |

### b) (Herstellung des Mischkristalls)

Eine Mischung von 6,69 g (12,0 mMol) des Produktes aus Beispiel 2a) und 6,46 g (12,0 mMol) des Produktes aus a) wird in 380 ml Toluol unter Rühren auf 60°C erhitzt Anschliessend werden 11,41 g Toluol-4-sulfonsäure zugegeben. Man erwärmt auf 100°C, rührt 1,5 Stunden bei dieser Temperatur und kühlt anschliessend auf Raumtemperatur ab. Man erhält 8,40 g eines purpurroten Pulvers.

| Analyse | C | H | N | Cl |
|---|---|---|---|---|
| Ber. | 65,76 % | 2,90 % | 12,20 % | 9,92 % |
| Gef. | 64,55 % | 3,12 % | 12,00 % | 9,62 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 15,5974 | 5,66 | 58 |
| 7,8658 | 11,24 | 25 |
| 6,5248 | 13,56 | 31 |
| 6,0649 | 14,59 | 36 |
| 3,8137 | 23,31 | 21 |
| 3,3093 | 26,92 | 100 |
| 2,9923 | 29,84 | 24 |

gekennzeichnet.

### c) (Herstellung der festen Lösung durch alkalische Umfällung)

Eine Suspension von 1,43 g (4,0 mMol) 1,4-Diketo-3,6-di-(4-chlorphenyl)-pyrrolo[3,4-c]pyrrol, 2,03 g (6,0 mMol) 1,4-Diketo-3,6-di-(4-cyanphenyl)-pyrrolo[3,4-c]pyrrol und 1,24 g Kaliumhydroxid in 75 ml Dimethylsulfoxid wird auf 50°C aufgewärmt und bei dieser Temperatur 2 Stunden gerührt. Anschliessend wird das Reaktionsgemisch auf Raumtemperatur abgekühlt und auf eine Mischung von 150 ml Wasser und 2,2 ml Salzsäure (rauchend, 37 %ig) gedrückt und dann bei Raumtemperatur 4 Stunden gerührt. Das rote Gemisch wird filtriert, mit Methanol und dann mit Wasser gewaschen und das Pigment bei 80°C im Vakuum getrocknet. Man erhält 3,2 g eines roten Pulvers.

| Analyse | C | H | N | Cl |
|---|---|---|---|---|
| Ber. | 66,68 % | 2,91 % | 12,96 % | 8,20 % |
| Gef. | 66,02 % | 3,01 % | 12,75 % | 8,02 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 16,0744 | 5,49 | 100 |
| 7,8749 | 11,23 | 30 |
| 6,5456 | 13,52 | 36 |
| 6,0875 | 14,54 | 48 |
| 3,7996 | 23,39 | 28 |
| 3,2872 | 27,11 | 100 |
| 3,0003 | 29,75 | 26 |

gekennzeichnet.

Daraus erkennt man, dass die Kristallstruktur dieser festen Lösung mit derjenigen des entsprechenden Mischkristalls (b) praktisch identisch ist

### Beispiel 5 a) (Herstellung des löslichen Diketopyrrolopyrrols)

Einer Suspension von 17,9 g 1,4-Diketo-3,6-di-(3-chlorphenyl)-pyrrolo[3,4-c]pyrrol in 500 ml Tetrahydrofuran werden 27,3 g Di-tert.-butyl-dicarbonat und danach 1,53 g 4-Dimethylaminopyridin zugegeben. Das Reaktionsgemisch wird 24 Stunden bei Raumtemperatur unter Ausschluss von atmosphärischer Feuchtigkeit gerührt. Danach wird das Lösungsmittel bei vermindertem Druck abdestilliert. Der Rückstand wird mit Methanol gewaschen und im Vakuum bei Raumtemperatur getrocknet. Man erhält 24,8 g (89 % d.Th.) N,N'-Di-tert.-butoxycarbonyl-1,4-diketo-3,6-di-(3-chlorphenyl)-pyrrolo[3,4-c]pyrrol in Form eines leuchtend gelben Produktes.

| Analyse | C | H | N | Cl |
|---|---|---|---|---|
| Ber. | 60,33 % | 4,70 % | 5,03 % | 12,72 % |
| Gef. | 60,33% | 4,79 % | 5,03 % | 12,72 % |

### b) (Herstellung des Mischkristalls aus den löslichen Diketopyrrolopyrrolen)

Eine Mischung von 4,89 g (10,0 mMol) des Produktes aus Beispiel 1a) und 5,57 g (10,0 mMol) des Produktes aus a) wird in 350 ml Toluol unter Rühren auf 60°C aufgewärmt. Zur entstandenen Lösung werden 9,51 g Toluol-4-sulfonsäure-monohydrat zugegeben und auf 105°C erhitzt, anschliessend lässt man auf Raumtemperatur abkühlen. Die gebildete feste Substanz wird abfiltriert, zuerst mit Methanol, dann mit Wasser gewaschen und im Vakuum bei 80°C getrocknet. Man erhält 6,1 g eines roten Pulvers.

| Analyse | C | H | N | Cl |
|---|---|---|---|---|
| Ber. | 66,99 % | 3,44 % | 8,68 % | 10,98 % |
| Gef. | 66,48 % | 3,42 % | 8,68 % | 11,25 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 14,3114 | 6,17 | 92 |
| 6,7532 | 13,10 | 52 |
| 6,4304 | 13,76 | 25 |
| 5,8638 | 15,10 | 25 |
| 4,7102 | 18,83 | 35 |
| 3,7250 | 23,87 | 33 |
| 3,4730 | 25,63 | 44 |
| 3,2671 | 27,27 | 100 |
| 2,3366 | 35,50 | 11 |

gekennzeichnet.

### c) (Herstellung des Mischkristalls durch alkalische Umfällung)

Eine Mischung aus 2,02 g (7,0 mMol) 1,4-Diketo-3,6-diphenyl-pyrrolo[3,4-c]pyrrol, 2,50 g (7,0 mMol) 1,4-Diketo-3,6-di-(3-chlorphenyl)-pyrrolo[3,4-c]pyrrol und 1,24 g Natriumhydroxid in 100 ml 1-Methyl-2-pyrrolidon wird 24 Stunden bei Raumtemperatur gerührt. Anschliessend wird das Reaktionsgemisch während 70 Minuten in eine Mischung von 100 ml Methanol, 100 ml Wasser und 1,67 ml konz. Schwefelsäure ausgetragen und dann bei Raumtemperatur 6 Stunden gerührt. Das rote Gemisch wird abfiltriert, mit Wasser gewaschen und bei 60°C im Vakuum getrocknet. Man erhält 4,1 g (87 % d.Th.) eines roten Pulvers.

| Analyse | C | H | N | Cl |
|---|---|---|---|---|
| Ber. | 66,99 % | 3,44 % | 8,68 % | 10,98 % |
| Gef. | 66,68 % | 3,47 % | 8,68 % | 10,84 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 14,2590 | 6,19 | 100 |
| 6,7650 | 13,08 | 41 |
| 6,4380 | 13,74 | 24 |
| 5,9045 | 14,99 | 22 |
| 4,7043 | 18,85 | 39 |
| 3,7186 | 23,91 | 32 |
| 3,4688 | 25,66 | 40 |
| 3,2725 | 27,23 | 89 |
| 2,3380 | 38,47 | 10 |

gekennzeichnet.

### d) (Herstellung der festen Lösung durch alkalische Umfällung)

Eine Mischung aus 1,73 g (6,0 mMol) 1,4-Diketo-3,6-diphenyl-pyrrolo[3,4-c]pyrrol, 1,43 g (4,0 mMol) 1,4-Diketo-3,6-di-(3-chlorphenyl)-pyrrolo[3,4-c]pyrrol und 0,88 g Natriumhydroxid in 75 ml 1-Methyl-2-pyrrolidon wird auf 50°C aufgewärmt und bei dieser Temperatur über Nacht gerührt. Anschliessend wird das Reaktionsgemisch auf Raumtemperatur abgekühlt und während 75 Minuten in eine Mischung von 75 ml Methanol, 75 ml Wasser und 1,2 ml konz. Schwefelsäure bei 10°C ausgetragen und dann bei Raumtemperatur 20 Stunden gerührt. Das rote Gemisch wird abfiltriert, mit Methanol und danach mit Wasser gewaschen und bei 60°C im Vakuum getrocknet Man erhält 2,7 g (85 % d.Th.) eines roten Pulvers.

| Analyse | C | H | N | Cl |
|---|---|---|---|---|
| Ber. | 69,20 % | 3,65 % | 8,97 % | 7,94 % |
| Gef. | 68,05 % | 3,64 % | 8,92 % | 8,60 % |

Das Röntgenbeugungsdiagramm ist durch folgende Beugungslinien

| Netzebenenabstände (d-Werte in Å) | doppelte Glanzwinkel (2Θ) | relative Intensität |
|---|---|---|
| 14,4039 | 6,13 | 100 |
| 6,7881 | 13,03 | 34 |
| 6,4211 | 13,78 | 22 |
| 5,9361 | 14,91 | 20 |
| 4,7087 | 18,83 | 34 |
| 3,7402 | 23,77 | 30 |
| 3,4720 | 25,64 | 32 |
| 3,2886 | 27,09 | 80 |
| 2,3381 | 38,47 | 10 |

gekennzeichnet.

Daraus erkennt man, dass die Kristallstruktur dieser festen Lösung mit derjenigen des entsprechenden Mischkristalls (b & c) praktisch identisch ist.

Beispiel 6: Beispiel 4 wird wiederholt mit der einzigen Ausnahme, dass anstelle des 1,4-Diketo-3,6-di-(4-cyanphenyl)-pyrrolo[3,4-c]pyrrols die äquivalente Menge 1,4-Diketo-3,6-diphenyl-pyrrolo[3,4-c]pyrrol eingesetzt wird. Man erhält ein Mischkristall dessen Kristallstruktur praktisch identisch ist mit derjenigen des entsprechenden asymmetrischen Diketopyrrolopyrrols der Formel

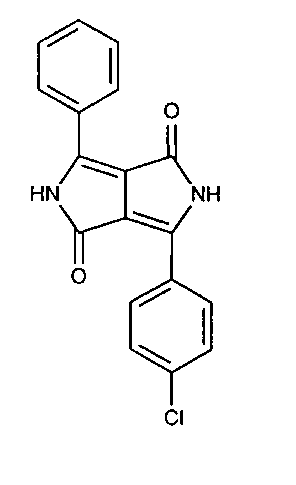

| Analyse | C | H | N | Cl |
|---|---|---|---|---|
| Ber. | 66,99 % | 3,44 % | 8,68 % | 10,98 % |
| Gef. | 66,36 % | 3,50 % | 8,63 % | 11,02 % |

Beispiel 7: Beispiel 1 wird wiederholt mit der einzigen Ausnahme, dass anstelle des 1,4-Diketo-3,6-di-(4-tert.-butylphenyl)-pyrrolo[3,4-c]pyrrols die äquivalente Menge 1,4-Diketo-3,6-di(3-cyanophenyl)-pyrrolo[3,4-c]pyrrol eingesetzt wird. Man erhält ein Mischkristall dessen Kristallstruktur praktisch identisch ist mit derjenigen des entsprechenden asymmetrischen Diketopyrrolopyrrols der Formel

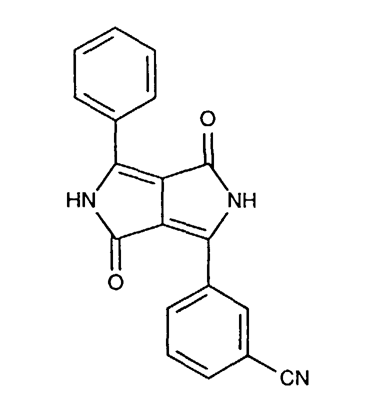

| Analyse | C | H | N |
|---|---|---|---|
| Ber. | 72,84 % | 3,54 % | 13,41 % |
| Gef. | 72,07 % | 3,63 % | 13,15 % |

Beispiel 8: Beispiel 1 wird wiederholt mit der einzigen Ausnahme, dass anstelle des 1,4-Diketo-3,6-di-(4-tert.-butylphenyl)-pyrrolo[3,4-c]pyrrols die äquivalente Menge 1,4-Diketo-3,6-di-(3,4-dichlorophenyl)-pyrrolo[3,4-c]pyrrol eingesetzt wird. Man erhält ein Mischkristall dessen Kristallstruktur praktisch identisch ist mit derjenigen des entsprechenden asymmetrischen Diketopyrrolopyrrols der Formel

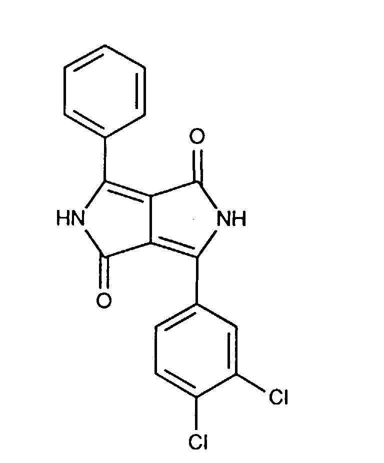

| Analyse | C | H | N | Cl |
|---|---|---|---|---|
| Ber. | 60,53 % | 2,82 % | 7,84 % | 19,85 % |
| Gef. | 60,47 % | 2,95 % | 7,83 % | 19,55 % |

Beispiel 9: Beispiel 4c) wird wiederholt mit der Ausnahme, dass anstelle von 4,0 mMol 1,4-Diketo-3,6-di-(4-chlorphenyl)-pyrrolo[3,4-c]pyrrol und 6,0 mMol 1,4-Diketo-3,6-di-(4-cyanphenyl)-pyrrolo[3,4-c]pyrrol 5,0 mMol 1,4-Diketo-3,6-di-(4-chlorphenyl)-pyrrolo[3,4-c]pyrrol und 5,0 mMol 1,4-Diketo-3,6-di-(3,4-dichlorphenyl)-pyrrolo-[3,4-c]pyrrol und anstelle von 1,24 g Kaliumhydroxid und 75 ml Dimethylsulfoxid 1,68 g Kaliumhydroxid und 70 ml Dimethylsulfoxid eingesetzt werden. Man erhält ein Mischkristall dessen Kristallstruktur praktisch identisch ist mit derjenigen des entsprechenden asymmetrischen Diketopyrrolopyrrols der Formel

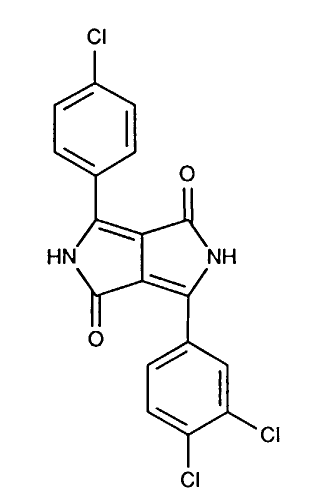

| Analyse | C | H | N | Cl |
|---|---|---|---|---|
| Ber. | 55,20 % | 2,32 % | 7,15 % | 27,16 % |
| Gef. | 55,14 % | 2,47 % | 7,11 % | 26,53 % |

Beispiel 10: Beispiel 1 wird wiederholt mit der einzigen Ausnahme, dass anstelle des 1,4-Diketo-3,6-di-(4-tert.-butylphenyl)-pyrrolo[3,4-c]pyrrols die äquivalente Menge 1,4-Diketo-3,6-di-(3-methylphenyl)-pyrrolo[3,4-c]pyrrol eingesetzt wird. Man erhält ein Mischkristall dessen Kristallstruktur praktisch identisch ist mit derjenigen des entsprechenden asymmetrischen Diketopyrrolopyrrols der Formel

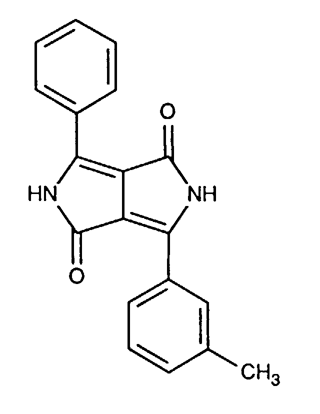

| Analyse | C | H | N |
|---|---|---|---|
| Ber. | 75,48 % | 4,67 % | 9,27 % |
| Gef. | 75,12 % | 4,75 % | 9,21 % |

Beispiel 11: Beispiel 1 wird wiederholt mit der einzigen Ausnahme, dass anstelle von 1,4-Diketo-3,6-diphenyl-pyrrolo[3,4-c]pyrrol und 1,4-Diketo-3,6-di-(4-tert.-butylphenyl)-pyrrolo[3,4-c]pyrrol die äquivalente Menge 1,4-Diketo-3,6-di-(4-chlorphenyl)-pyrrolo-[3,4-c]pyrrol und 1,4-Diketo-3,6-di-(4-methylphenyl)-pyrrolo[3,4-c]pyrrol eingesetzt wird. Man erhält ein Mischkristall dessen Kristallstruktur praktisch identisch ist mit derjenigen des entsprechenden asymmetrischen Diketopyrrolopyrrols der Formel

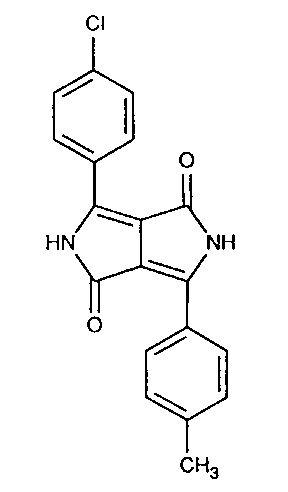

| Analyse | C | H | N | Cl |
|---|---|---|---|---|
| Ber. | 67,76 % | 3,89 % | 8,32 % | 10,53 % |
| Gef. | 67,52 % | 4,00 % | 8,26 % | 10,68 % |

Beispiel 12: Beispiel 11 wird wiederholt mit der einzigen Ausnahme, dass anstelle des 1,4-Diketo-3,6-di-(4-methylphenyl)-pyrrolo[3,4-c]pyrrols die äquivalente Menge 1,4-Diketo-3,6-di(3-cyanophenyl)-pyrrolo[3,4-c]pyrrol eingesetzt wird. Man erhält ein Mischkristall dessen Kristallstruktur praktisch identisch ist mit derjenigen des entsprechenden asymmetrischen Diketopyrrolopyrrols der Formel

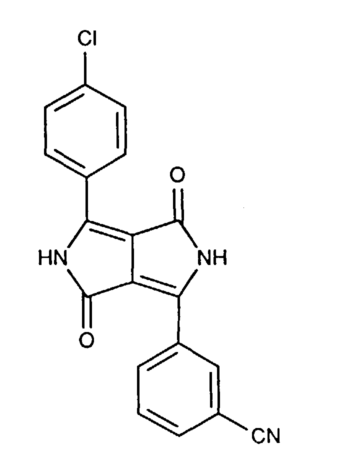

| Analyse | C | H | N | Cl |
|---|---|---|---|---|
| Ber. | 65,76 % | 2,90 % | 12,20 % | 9,92 % |
| Gef. | 65,16 % | 3,17 % | 11,88 % | 10,06 % |

### Beispiel 13:

| | |
|---|---|
| 7,5 g | des Mischkristalls aus Beispiel 1, 98,9 g CAB-Lösung bestehend aus |
| 41,0 g | Celluloseacetobutyrat ®CAB 531.1, 20 %ig in Butanol/Xylol 2:1 (Eastman Chem.) |
| 1,5 g | Zirkonium Octoat, |
| 18,5 g | ®SOLVESSO 150* (ESSO), |
| 21,5 g | Butylacetat und |
| 17,5 g | Xylol, |

36,5 g Polyesterharz ®DYNAPOL H700 (Dynamit Nobel), 4,6 g Melaminharz MAPRENAL MF650 (Hoechst) und 2,5 g Dispergiermitel ®DISPERBYK 160 (Byk Chemie) werden zusammen während 90 Minuten mit einer Schüttelmaschine dispergiert (Total Lack 150 g; 5 % Pigment).

27,69 g des so erhaltenen Volltonlacks werden für die Base-coat-Lackierung mit 17,31 g Al-Stammlösung (8 %ig) bestehend aus

| | |
|---|---|
| 12,65 g | ®SILBERLINE SS 3334AR, 60 %ig (Silberline Ltd.) |
| 56,33 g | CAB Lösung (Zusammensetzung wie oben) |
| 20,81 g | Polyesterharz ®DYNAPOL H700 |
| 2,60 g | Melaminharz ®MAPRENAL MF650 |
| 7,59 g | ®SOLVESSO 150 |

gemischt und auf ein Aluminiumblech spritzappliziert (Nassfilm ca. 20 µm). Nach einer Abdunstzeit von 30 Minuten bei Raumtemperatur wird ein TSA-Lack bestehend aus

| | |
|---|---|
| 29,60 g | Acrylharz ®URACRON 2263 XB, 50 %ig in Xylol/Butanol (Chem. Fabrik Schweizerhalle), |
| 5,80 g | Melaminharz ®CYMEL 327, 90 %ig in Isobutanol, |
| 2,75 g | Butylglycolacetat, |
| 5,70 g | Xylol, |
| 1,65 g | n-Butanol |
| 0,50 g | Siliconöl, 1 %ig in Xylol, |
| 3,00 g | Lichtschutzmittel ®TINUVIN 900, 10 %ig in Xylol (Ciba) |
| 1,00 g | Lichtschutzmittel ®TINUVIN 292, 10 %ig in Xylol (Ciba) |

als Top-coat-Lackierung spritzappliziert (Nassfilm ca. 50 µm). Anschliessend wird der Lack nach weiteren 30 Minuten Abdunsten bei Raumtemperatur, 30 Minuten bei 130°C eingebrannt.

Beispiel 14: 0,6 g des Mischkristalls von Beispiel 5c werden mit 67 g Polyvinylchlorid, 33 g Dioctylphthalat, 2 g Dibutylzinndilaurat und 2 g Titandioxid vermischt und auf einem Walzenstuhl während 15 Minuten bei 160°C zu einer dünnen Folie verarbeitet. Die so erzeugte rote PVC-Folie ist sehr farbstark, migrations- und lichtbeständig.

Beispiel 15: 1000 g Polypropylengranulat (®DAPLEN PT-55, Chemie LINZ) und 20 g eines 50 %-igen Pigmentpräparates, bestehend aus 10 g der festen Lösung von Beispiel 3b und 10 g Mg-Behenat, werden in einer Mischtrommel intensiv vermischt. Das so behandelte Granulat wird bei 260 bis 285°C nach dem Schmelzspinnverfahren versponnen. Man erhält rotgefärbte Fasern mit sehr guten Licht- und textilen Echtheiten.

## Patentansprüche

1. Mischkristalle von 1,4-Diketopyrrolo[3,4-c]pyrrolen, bestehend aus zwei unterschiedlichen Verbindungen der Formeln 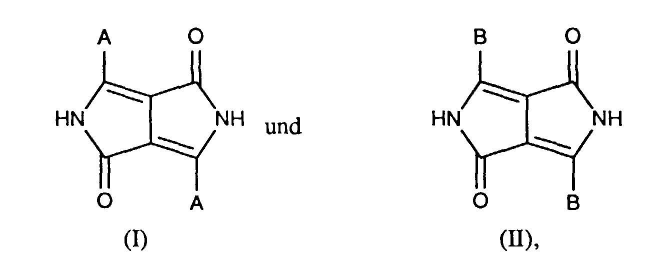 im Molverhältnis 1:1, worin A und B, die verschieden sein müssen, jeweils für eine Gruppe der Formel 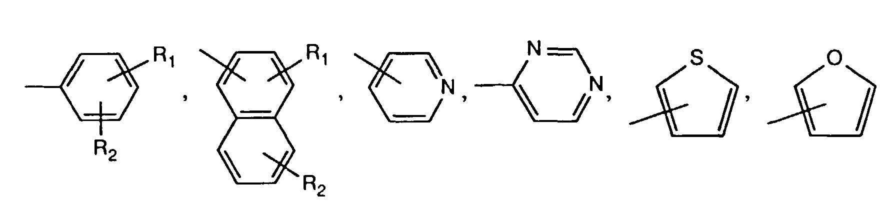 oder 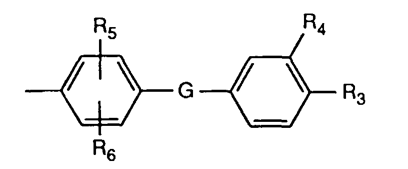 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, C₁-C₁₈-Alkoxycarbonyl, C₁-C₁₈-Alkylaminocarbonyl, -CN, -NO₂, Trifluormethyl, C₅-C₆-Cycloalkyl, -C=N-(C₁-C₁₈-Alkyl), 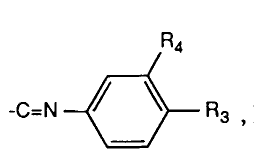 Imidazolyl, Pyrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten, G -CH₂-, -CH(CH₃)-, -C(CH₃)₂-, -CH=N-, -N=N-, -O-, -S-, -SO-, -SO₂-, -CONH- oder -NR₇- ist,
R₃ und R₄ unabhängig voneinander Wasserstoff, Halogen, C₁-C₆-Alkyl, C₁-C₁₈Alkoxy oder -CN sind, R₅ und R₆ unabhängig voneinander Wasserstoff, Halogen oder C₁-C₆-Alkyl und R₇ Wasserstoff oder C₁-C₆-Alkyl bedeuten.

2. Mischkristalle gemäss Anspruch 1, dadurch gekennzeichnet, dass in den Formeln I und II A und B jeweils eine Gruppe der Formel 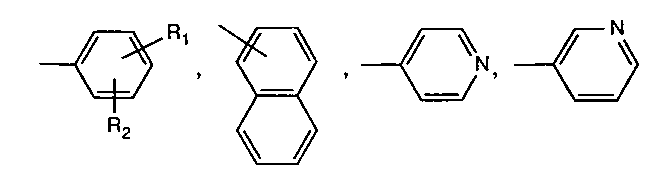 oder 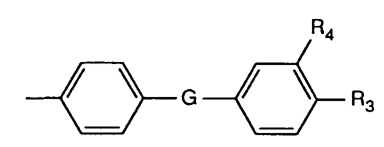 sind,
worin
R₁ und R₂ unabhängig voneinander Wasserstoff, Chlor, Brom, C₁-C₄-Alkyl, C₁-C₆-Alkoxy, C₁-C₆-Alkylamino oder CN bedeuten,
G -O-, -NR₇-, -N=N- oder -SO₂- ist,
R₃ und R₄ Wasserstoff und R₇ Wasserstoff, Methyl oder Ethyl bedeuten.

3. Mischkristalle gemäss Anspruch 2, dadurch gekennzeichnet, dass in den Formeln I und II A und B jeweils eine Gruppe der Formel 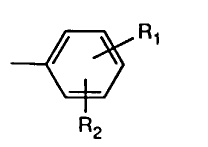 sind, worin R₁ und R₂ unabhängig voneinander Wasserstoff, Methyl, tert.-Butyl, Chlor, Brom oder CN bedeuten.

4. Mischkristalle gemäss Anspruch 3, dadurch gekennzeichnet, dass R₂ Wasserstoff ist.

5. Verfahren zur Herstellung von Mischkristallen gemäss Anspruch 1, dadurch gekennzeichnet, dass die Verbindungen der Formeln I und II nach an sich bekannten Methoden mit einem Dicarbonat der Formel
D-O-D (III)
oder mit einem Trihaloessigsäureester der Formel
(R₈)₃C - D (IV),
oder mit einem Azid der Formel
DN₃ (V),
oder mit einem Carbonat der Formel
D-OR₉ (VI),
oder mit einem Alkylideniminooxyameisensäureester der Formel 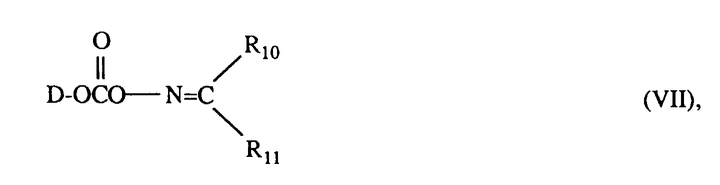 worin D eine Gruppe der Formel 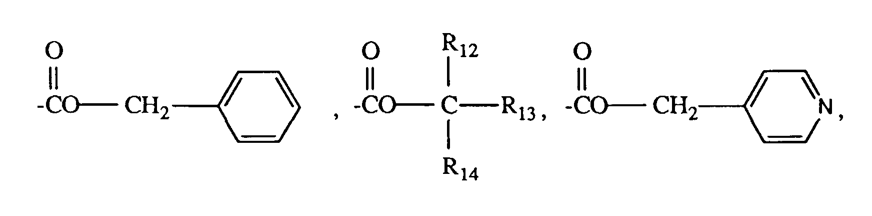 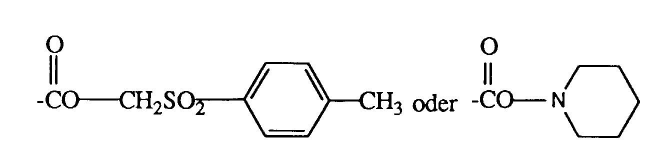 bedeutet,
R₈ Chlor, Fluor oder Brom, R₉ C₁-C₄-Alkyl oder unsubstituiertes oder durch Halogen, C₁-C₄-Alkyl, C₁-C₄-Alkoxy oder -CN substituiertes Phenyl, R₁₀ -CN oder -COOR₉ und R₁₁ unsubstituiertes oder durch Halogen, C₁-C₄-Alkyl, C₁-C₄-Alkoxy oder -CN substituiertes Phenyl sind und R₁₂, R₁₃ und R₁₄ unabhängig voneinander Wasserstoff, C₁-C₆-Alkyl oder C₂-C₅-Alkenyl bedeuten, wobei mindestens zwei von R₁₂, R₁₃ und R₁₄ Alkyl oder Alkenyl sein müssen, im Molverhältnis 1:2 in einem aprotischen organischen Lösungsmittel in Gegenwart einer Base als Katalysator, in lösliche Verbindungen der Formeln 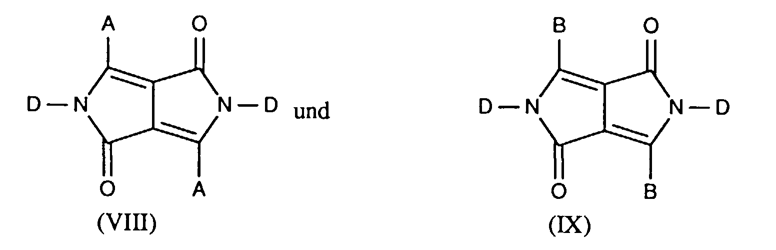 umgesetzt werden,
- diese Verbindungen entweder
a) nach allgemein bekannten Methoden in Pulverform im Molverhältnis 1:1 homogen vermischt werden oder
b) nach allgemein bekannten Methoden in Pulverform im Molverhältnis 1:1 homogen vermischt werden und das Gemisch in einem Lösungsmittel gelöst wird oder
c) zuerst gelöst werden und in Lösung im Mischverhältnis 1:1 vermischt werden
und danach
- aus dem trockenen oder gelösten Gemisch durch thermische, photolytische oder chemische Behandlung der gewünschte Mischkristall ausgefällt wird.

6. Verfahren gemäss Anspruch 5, dadurch gekennzeichnet, dass in den Formeln III-VII D eine Gruppe der Formel 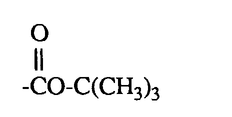 bedeutet.

7. Feste Lösungen von 1,4-Diketopyrrolo[3,4-c]pyrrolen, bestehend aus zwei unterschiedlichen Verbindungen der Formeln 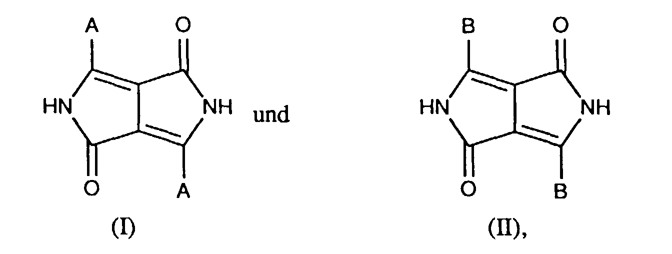 worin A und B, die verschieden sein müssen, jeweils für eine Gruppe der Formel 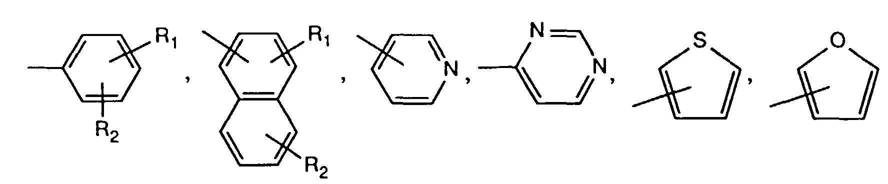 oder 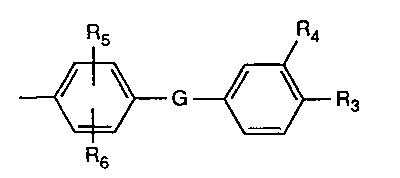 stehen, worin
R₁ und R₂ unabhängig voneinander Wasserstoff, Halogen, C₁-C₁₈-Alkyl, C₁-C₁₈-Alkoxy, C₁-C₁₈-Alkylmercapto, C₁-C₁₈-Alkylamino, -CN, -NO₂, Trifluormethyl, C₅-C₆-Cycloalkyl, -C=N-(C₁-C₁₈-Alkyl), 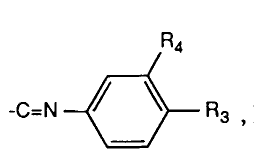 Imidazolyl, Pyrazolyl, Triazolyl, Piperazinyl, Pyrrolyl, Oxazolyl, Benzoxazolyl, Benzthiazolyl, Benzimidazolyl, Morpholinyl, Piperidinyl oder Pyrrolidinyl bedeuten,
G -CH₂-, -CH(CH₃)-, -C(CH₃)₂-, -CH=N-, -N=N-, -O-, -S-, -SO-, -SO₂- oder -NR₇- ist,
R₃ und R₄ unabhängig voneinander Wasserstoff, Halogen, C₁-C₆-Alkyl, C₁-C₁₈Alkoxy oder -CN sind, R₅ und R₆ unabhängig voneinander Wasserstoff, Halogen oder C₁-C₆-Alkyl und R₇ Wasserstoff oder C₁-C₆-Alkyl bedeuten,
mit der Massgabe, dass das die kleinere geometrische Konstitution aufweisende 1,4-Diketopyrrolo[3,4-c]pyrrol in einer Menge von 50 bis 70 Mol%, bevorzugt 55 bis 60 Mol%, enthalten ist.

8. Feste Lösungen gemäss Anspruch 7, dadurch gekennzeichnet, dass in den Formeln I und II A und B jeweils eine Gruppe der Formel 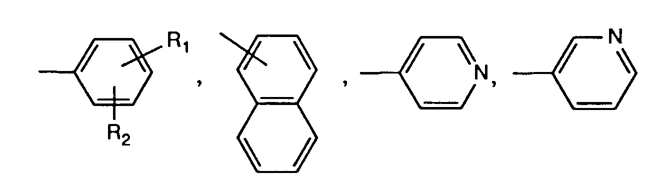 oder 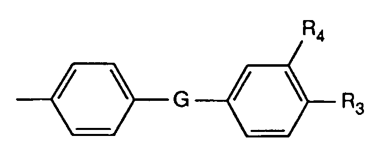 sind,
worin
R₁ und R₂ unabhängig voneinander Wasserstoff, Chlor, Brom, C₁-C₄-Alkyl, C₁-C₆-Alkoxy, C₁-C₆-Alkylamino, CN oder Phenyl bedeuten,
G -O-, -NR₇-, -N=N- oder -SO₂- ist,
R₃ und R₄ Wasserstoff und R₇ Wasserstoff, Methyl oder Ethyl bedeuten.

9. Feste Lösungen gemäss Anspruch 8, dadurch gekennzeichnet, dass in den Formeln I und II A und B jeweils eine Gruppe der Formel 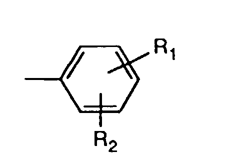 sind, worin R₁ und R₂ unabhängig voneinander Wasserstoff, Methyl, tert.-Butyl, Chlor, Brom oder CN bedeuten.

10. Hochmolekulares organisches Material enthaltend einen Mischkristall gemäss Anspruch 1.

11. Hochmolekulares organisches Material gemäss Anspruch 10, dadurch gekennzeichnet, dass es ein Kunststoff ist.

12. Hochmolekulares organisches Material gemäss Anspruch 10, dadurch gekennzeichnet, dass es ein Lack ist.

13. Hochmolekulares organisches Material enthaltend eine feste Lösung gemäss Anspruch 7.

14. Hochmolekulares organisches Material gemäss Anspruch 13, dadurch gekennzeichnet, dass es ein Kunststoff ist.

15. Hochmolekulares organisches Material gemäss Anspruch 13, dadurch gekennzeichnet, dass es ein Lack ist.

## Claims

1. Mixed crystals of 1,4-diketopyrrolo[3,4-c]-pyrroles, consisting of two different compounds of formulae 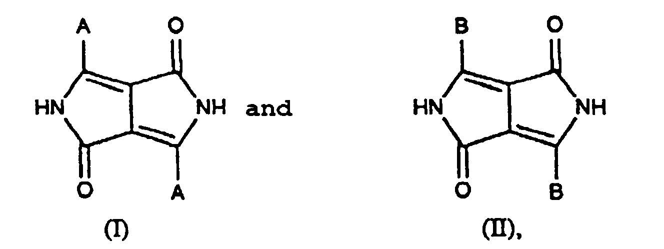 in the molar ratio of 1:1, wherein A and B, which must be different, are each a group of formula 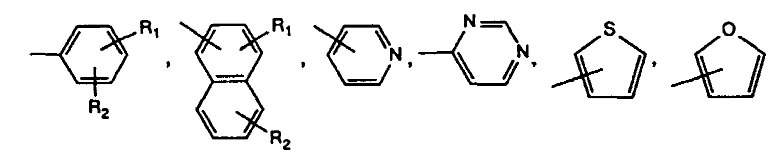 or 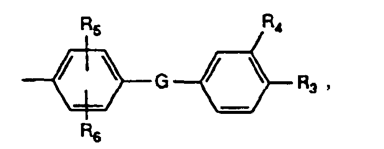 wherein
R₁ and R₂ are each independently of the other hydrogen, halogen, C₁-C₁₈alkyl, C₁-C₁₈alkoxy, C₁-C₁₈alkylmercapto, C₁-C₁₈alkylamino, C₁-C₁₈alkoxycarbonyl, C₁-C₁₈alkyl-aminocarbonyl, -CN, -NO₂, trifluoromethyl, C₅-C₆cycloalkyl,
-C=N- (C₁-C₁₈alkyl), 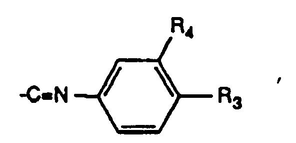 imidazolyl, pyrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂-, -CONH- or -NR₇-,
R₃ and R₄ are each independently of the other hydrogen, halogen, C₁-C₆alkyl, C₁-C₁₈alkoxy or -CN, R₅ and R6 are each independently of the other hydrogen, halogen or C₁-C₆alkyl, and R₇ is hydrogen or C₁-C₆alkyl.

2. Mixed crystals according to claim 1, wherein A and B in formulae I and II are each a group of formula 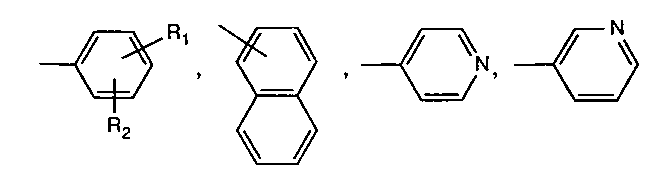 or 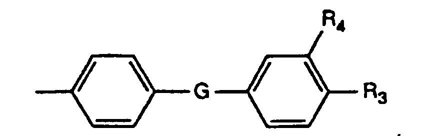 wherein
R₁ and R₂ are each independently of the other hydrogen, chloro, bromo, C₁-C₄alkyl, C₁-C₆alkoxy, C₁-C₆alkylamino or CN,
G is -O-, -NR₇-, -N=N- or -SO₂-,
R₃ and R₄ are hydrogen, and R₇ is hydrogen, methyl or ethyl.

3. Mixed crystals according to claim 2, wherein A and B in formulae I and II are each a group of formula 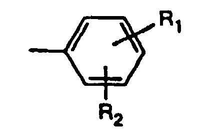 wherein R₁ and R₂ are each independently of the other hydrogen, methyl, tert-butyl, chloro, bromo or CN.

4. Mixed crystals according to claim 3, wherein R₂ is hydrogen.

5. A process for the preparation of mixed crystals according to claim 1, which comprises the compounds of formulae I and II being reacted by methods known per se with a dicarbonate of formula
D - O - D (III),
or with a trihaloacetate of formula
(R₈)₃C - D (IV),
or with an azide of formula
DN₃ (V),
or with a carbonate of formula
D-OR₉ (VI),
or with an alkylidene-iminooxyformate of formula 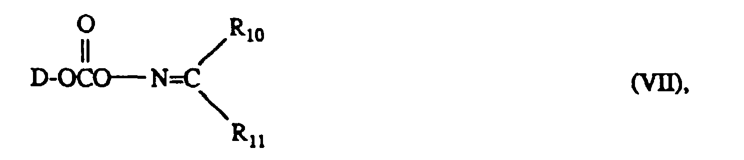 wherein D is a group of formula 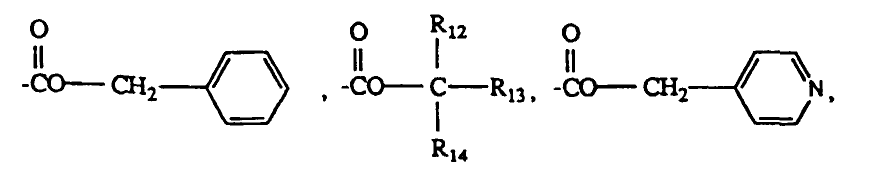 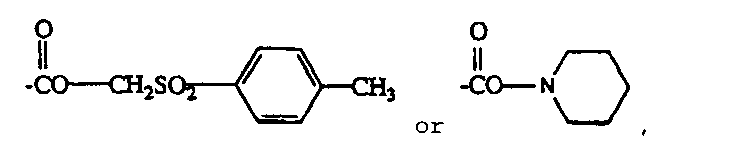 R₈ is chloro, fluoro or bromo, R₉ is C₁-C₄alkyl or is phenyl which is unsubstituted or substituted by halogen, C₁-C₄alkyl, C₁-C₄alkoxy or -CN, R₁₀ is -CN or -COOR₉, and R₁₁ is phenyl which is unsubstituted or substituted by halogen, C₁-C₄alkyl, C₁-C₄alkoxy or -CN, R₁₂, R₁₃ and R₁₄ are each independently of one another hydrogen, C₁-C₆alkyl or C₂-C₅alkenyl, and at least two of R₁₂, R₁₃ and R₁₄ must be alkyl or alkenyl, in the molar ratio of 1:2 in an aprotic organic solvent in the presence of a base as catalyst, to give soluble compounds of formulae 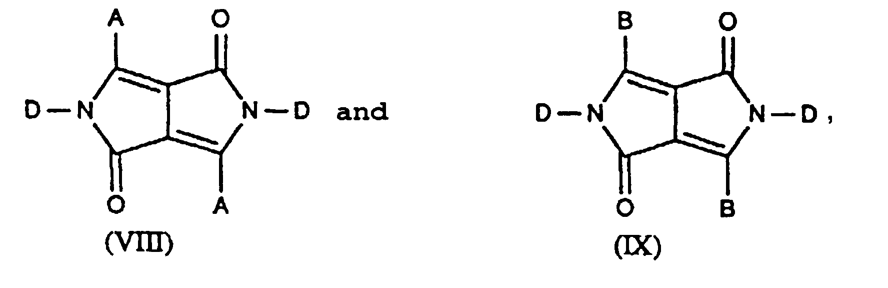
- and these compounds being either
a) mixed homogeneously in powder form by generally known methods in the molar ratio of 1:1, or
b) mixed homogeneously in powder form by generally known methods in the molar ratio of 1:1 and the mixture being dissolved in a solvent, or
c) first dissolved and then mixed in solution in the mixture ratio of 1:1,
and subsequently
- the desired mixed crystal being precipitated from the dry or dissolved mixture by thermal, photolytic or chemical treatment.

6. A process according to claim 5, wherein in formulae III-VII
D is a group of formula

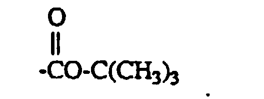

7. A solid solution of 1,4-diketopyrrolo-[3,4-c]pyrroles, consisting of two different compounds of formulae 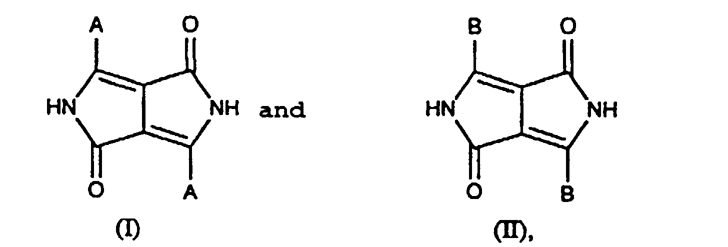 wherein A and B, which must be different, are each a group of formula 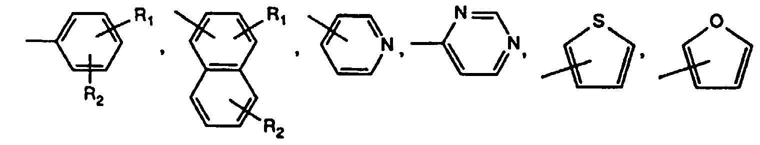 or 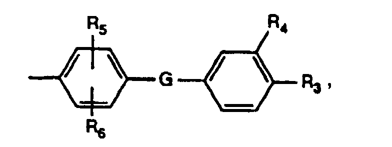 wherein
R₁ and R₂ are each independently of the other hydrogen, halogen, C₁-C₁₈alkyl, C₁-C₁₈alkoxy, C₁-C₁₈alkylmercapto, C₁-C₁₈alkylamino, -CN, -NO₂, trifluoromethyl, C₅-C₆cycloalkyl, -C=N- (C₁-C₁₈alkyl), 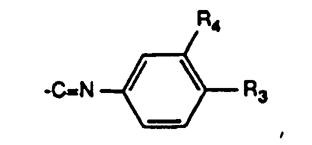 imidazolyl, pyrazolyl, triazolyl, piperazinyl, pyrrolyl, oxazolyl, benzoxazolyl, benzothiazolyl, benzimidazolyl, morpholinyl, piperidinyl or pyrrolidinyl,
G is -CH₂-, -CH(CH₃)-, -C(CH₃)₂-, -CH=N-, -N=N-, -O-, -S-, -SO-, -SO₂- or -NR₇-,
R₃ and R₄ are each independently of the other hydrogen, halogen, C₁-C₆alkyl, C₁-C₁₈alkoxy or -CN, R₅ and R₆ are each independently of the other hydrogen, halogen or C₁-C₆alkyl, and R₇ is hydrogen or C₁-C₆alkyl,
with the proviso that the 1,4-diketopyrrolo[3,4-c]-pyrrole having the smaller geometrical constitution is present in an amount of 50 to 70 mol%, preferably of 55 to 60 mol%.

8. A solid solution according to claim 7, wherein A and B in formulae I and II are each a group of formula 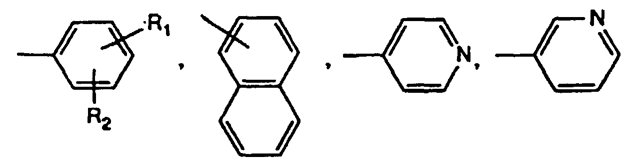 or 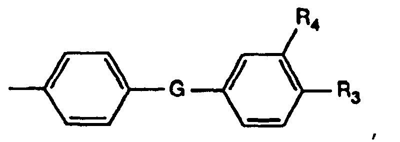 wherein
R₁ and R₂ are each independently of the other hydrogen, chloro, bromo, C₁-C₄alkyl, C₁-C₆alkoxy, C₁-C₆alkylamino, CN or phenyl,
G is -O-, -NR₇-, -N=N- or -SO₂-,
R₃ and R₄ are hydrogen, and R₇ is hydrogen, methyl or ethyl.

9. A solid solution according to claim 8, wherein A and B in formulae I and II are each a group of formula 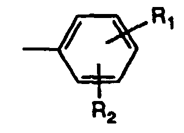 wherein R₁ and R₂ are each independently of the other hydrogen, methyl, tert-butyl, chloro, bromo or CN.

10. High molecular weight organic material comprising a mixed crystal according to claim 1.

11. High molecular weight organic material according to claim 10, which is a plastic.

12. High molecular weight organic material according to claim 10, which is a varnish.

13. High molecular weight organic material comprising a solid solution according to claim 7.

14. High molecular weight organic material according to claim 13, which is a plastic.

15. High molecular weight organic material according to claim 13, which is a varnish.

## Revendications

1. Cristaux mixtes des 1,4-dicéto-pyrrolo[3,4-c]-pyrroles, constitués par deux composés différents de formules 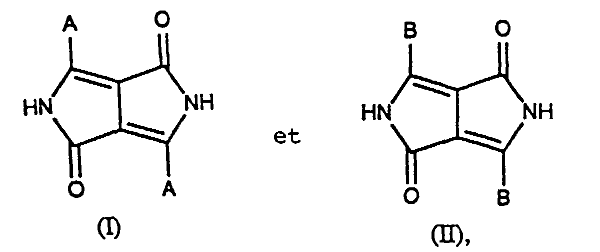 dans un rapport molaire 1:1, où A et B, lesquels doivent être différents, représentent chacun un groupe de formule 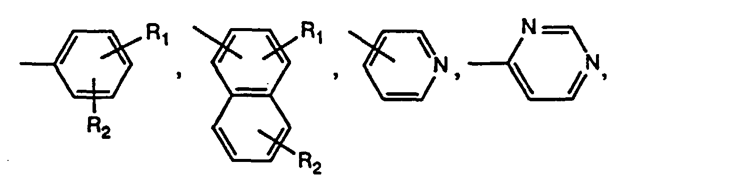 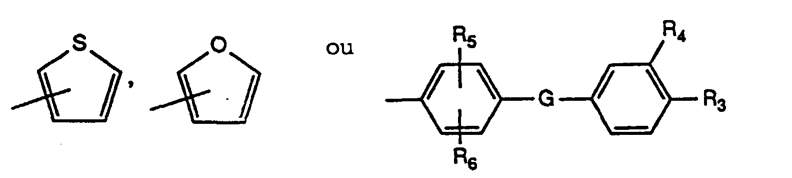 où
R₁ et R₂ représentent, indépendamment l'un de l'autre, des atomes d'hydrogène, des atomes d'halogène, des groupes alkyle en C₁-C₁₈, alkoxy en C₁-C₁₈, (alkyl en C₁-C₁₈)mercapto, (alkyl en C₁-C₁₈)amino, (alkoxy en C₁-C₁₈)carbonyle, (alkyl en C₁-C₁₈)amino-carbonyle, -CN, -NO₂, trifluorométhyle, cycloalkyle en C₅-C₆, -C=N(alkyle en C₁-C₁₈), 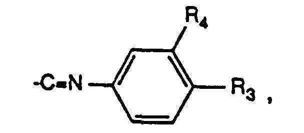 imidazolyle, pyrazolyle, triazolyle, pipérazinyle, pyrrolyle, oxazolyle, benzoxazolyle, benzthiazolyle, benzimidazolyle, morpholinyle, pipéridinyle ou pyrrolidinyle,
G représente -CH₂-, -CH(CH₃)-, -CH(CH₃)₂-, -CH=N-, -N=N-, -O-, -S-, -SO-, -SO₂-, -CONH- ou -NR₇-,
R₃ et R₄ représentent, indépendamment l'un de l'autre, des atomes d'hydrogène, des atomes d'halogène, des groupes alkyle en C₁-C₆, alkoxy en C₁-C₁₈ ou -CN,
R₅ et R₆ représentent, indépendamment l'un de l'autre, des atomes d'hydrogène, des atomes d'halogènes ou des groupes alkyle en C₁-C₆, et
R₇ représente un atome d'hydrogène ou un groupe alkyle en C₁-C₆.

2. Cristaux mixtes selon la revendication 1, caractérisés en ce que dans les formules I et II, A et B représentent chacun un groupe de formule 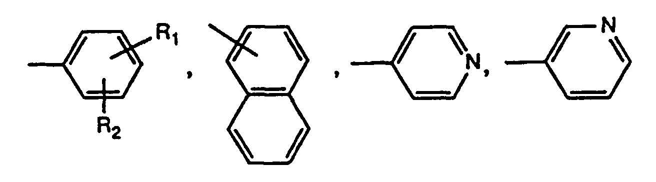 ou 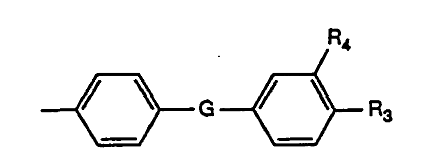 dans laquelle
R₁ et R₂ représentent, indépendamment l'un de l'autre, des atomes d'hydrogène, de chlore, de brome, des groupes alkyle en C₁-C₄, alkoxy en C₁-C₆, (alkyl en C₁-C₆)amino ou CN,
G représente -O-, -NR₇-, -N=N- ou -SO₂-,
R₃ et R₄ représentent des atomes d'hydrogène et
R₇ représente un atome d'hydrogène, des groupes méthyle ou éthyle.

3. Cristaux mixtes selon la revendication 2, caractérisés en ce que dans les formules I et II, A et B représentent chacun un groupe de formule 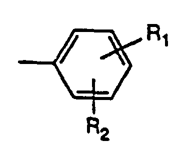 dans laquelle
R₁ et R₂ représentent, indépendamment l'un de l'autre, des atomes d'hydrogène, des groupes méthyle, tert.-butyle, des atomes de chlore, de brome ou CN.

4. Cristaux mixtes selon la revendication 3, caractérisés en ce que R₂ représente un atome d'hydrogène.

5. Procédé de préparation de cristaux mixtes selon la revendication 1, caractérisé en ce que l'on fait réagir les composés de formules I et II par des méthodes connues en soi, avec un dicarbonate de formule
D-O-D (III)
ou avec un ester d'acide trihaloacétique de formule
(R₈)₃C-D (IV),
ou avec un azoture de formule
DN₃ (V),
ou avec un carbonate de formule
D-OR₉ (VI),
ou avec un ester d'un acide alkylidène iminooxyformique de formule 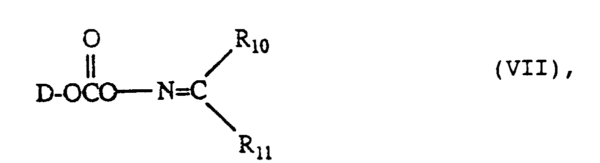 dans laquelle D représente un groupe de formule 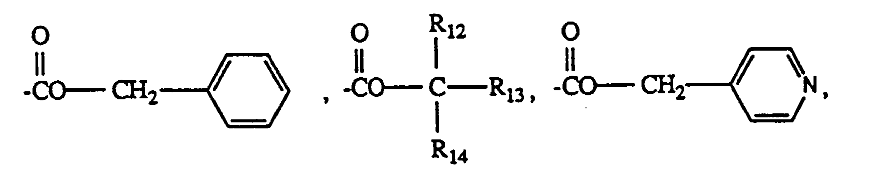 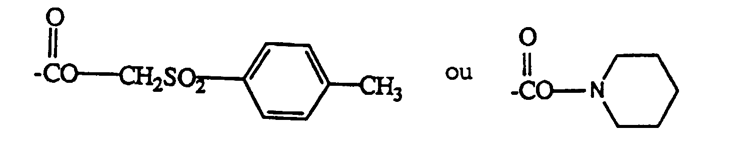
R₈ représente des atomes de chlore, de fluor ou de brome,
R₉ représente des groupes alkyle en C₁-C₄ ou phényle non substitué ou substitué par des substituants halogène, alkyle en C₁-C₄, alkoxy en C₁-C₄ ou -CN,
R₁₀ représente -CN ou -COOR₉, et
R₁₁ représente phényle non substitué ou substitué par des substituants halogène, alkyle en C₁-C₄, alkoxy en C₁-C₄ ou -CN, et
R₁₂, R₁₃ et R₁₄ représentent, indépendamment les uns des autres, des atomes d'hydrogène, des groupes alkyle en C₁-C₆ ou alcényle en C₂-C₅, au moins deux de R₁₂, R₁₃ et R₁₄ doivent être alkyle ou alcényle,
dans un rapport molaire 1:2 dans un solvant organique protique en présence d'une base comme catalyseur, pour donner des composés solubles de formules 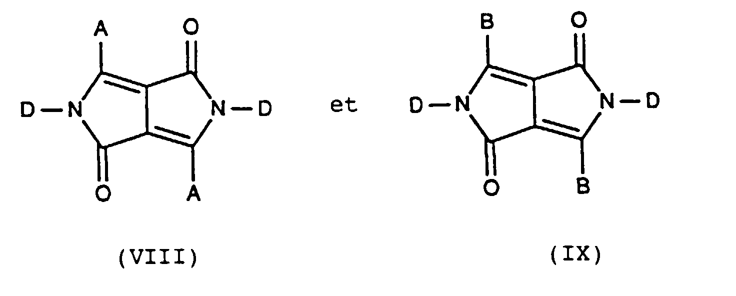
- ces composés sont
a) soit mélangés selon des méthodes usuelles sous forme pulvérulente dans un rapport molaire 1:1 pour donner un mélange homogène,
b) ou mélangés selon des méthodes usuelles sous forme pulvérulente dans un rapport molaire 1:1 pour donner un mélange homogène et le mélange est dissous dans un solvant,
c) soit d'abord dissous et mélangés en solution dans un rapport molaire 1:1,
et ensuite
- le cristal mixte voulu est précipité à partir du mélange sec ou dissous par traitement thermique, photolytique ou chimique.

6. Procédé selon la revendication 5, caractérisé en ce que dans les formules III-VII, D représente un groupe de formule

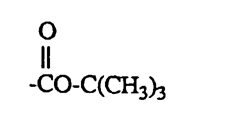

7. Solutions solides de 1,4-dicéto-pyrrolo[3,4-c]-pyrroles constituées par des composés différents de formules 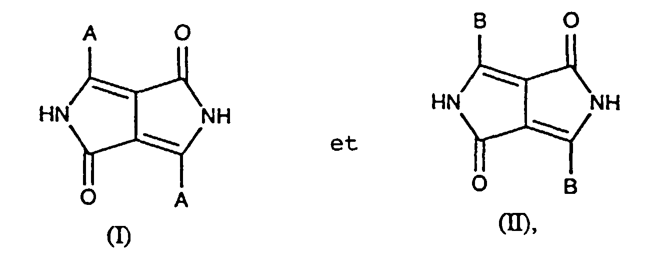 dans un rapport molaire 1:1, où A et B, lesquels doivent être différents, représente chacun un groupe de formule 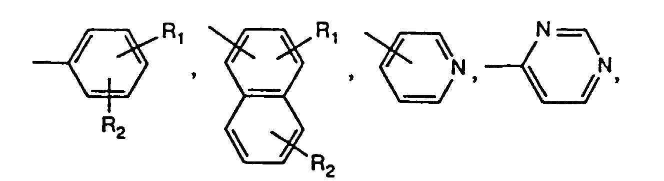 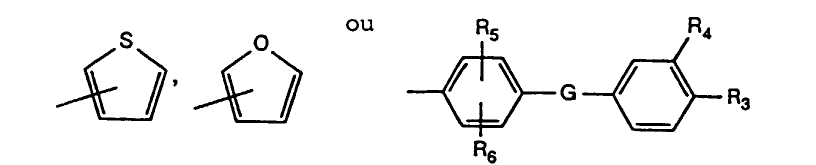 où
R₁ et R₂ représentent, indépendamment l'un de l'autre, des atomes d'hydrogène, des atomes d'halogènes, des groupes alkyle en C₁-C₁₈, alkoxy en C₁-C₁₈, (alkyl en C₁-C₁₈)mercapto, (alkyl en C₁-C₁₈)amino, -CN, -NO₂, trifluorométhyle, cycloalkyle en C₅-C₆, C=N(alkyle en C₁-C₁₈), 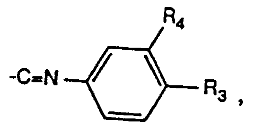 imidazolyle, pyrazolyle, triazolyle, pipérazinyle, pyrrolyle, oxazolyle, benzoxazolyle, benzthiazolyle, benzimidazolyle, morpholinyle, pipéridinyle ou pyrrolidinyle,
G représente -CH₂-, -CH(CH₃)-, -CH(CH₃)₂-, -CH=N-, -N=N-, -O-, -S-, -SO-, -SO₂-, ou -NR₇-,
R₃ et R₄ représentent, indépendamment l'un de l'autre, des atomes d'hydrogène, des atomes d'halogènes, des groupes alkyle en C₁-C₆, alkoxy en C₁-C₁₈ ou -CN,
R₅ et R₆ représentent, indépendamment l'un de l'autre, des atomes d'hydrogène, des atomes d'halogènes ou des groupes alkyle en C₁-C₆, et
R₇ représente un atome d'hydrogène ou un groupe alkyle en C₁-C₆,
à la condition que le 1,4-dicéto-pyrrolo[3,4-c]-pyrrole présentant la constitution géométrique plus petite soit contenu en une quantité de 50 à 70% en moles, de préférence de 55 à 60% en moles.

8. Solutions solides selon la revendication 7, caractérisées en ce que, dans les formules I et II, A et B représentent chacun un groupe de formule 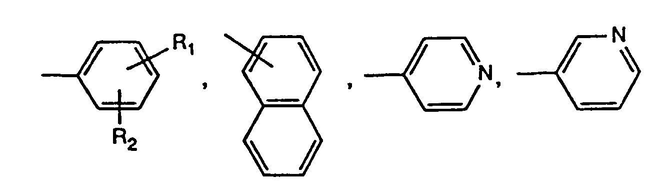 ou 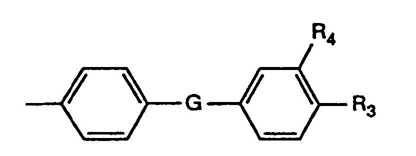 où
R₁ et R₂ représentent, indépendamment l'un de l'autre, des atomes d'hydrogène, de chlore, de brome, des groupes alkyle en C₁-C₄, alkoxy en C₁-C₆, (alkyl en C₁-C₆)amino, CN ou phényle,
G représente -O-, -NR₇-, -N=N- ou SO₂-,
R₃ et R₄ représentent des atomes d'hydrogène, des groupes méthyle ou éthyle.

9. Solutions solides selon la revendication 8, caractérisées en ce que, dans les formules I et II, A et B représentent chacun un groupe de formule 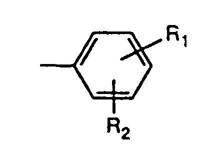 dans laquelle
R₁ et R₂ représentent, indépendamment l'un de l'autre, des atomes d'hydrogène, des groupes méthyle, tert.-butyle, des atomes de chlore, de brome ou -CN.

10. Matière organique de haut poids moléculaire contenant au moins un cristal mixte selon la revendication 1.

11. Matière organique de haut poids moléculaire selon la revendication 10, caractérisée en ce qu'il s'agit d'une matière plastique.

12. Matière organique de haut poids moléculaire selon la revendication 10, caractérisée en ce qu'il s'agit d'un vernis.

13. Matière organique de haut poids moléculaire contenant une solution solide selon la revendication 7.

14. Matière organique de haut poids moléculaire selon la revendication 13, caractérisée en ce qu'il s'agit d'une matière plastique.

15. Matière organique de haut poids moléculaire selon la revendication 13, caractérisée en ce qu'il s'agit d'un vernis.
